# EUROPEAN PATENT SPECIFICATION

(11) **EP 4 244 387 B1**
(45) Date of publication and mention of the grant of the patent: **17.06.2026**
(21) Application number: 21815389.8
(22) Date of filing: 12.11.2021
(51) Int. Cl.: C12Q 1/6869, G01N 21/552, G01N 21/64

(54) **ALTERING FLOW CELL SIGNALS**
VERÄNDERUNG DER FLUSSZELLENSIGNALE
MODIFICATION DES SIGNAUX DE CELLULES DE FLUX

(30) Priority: 16.11.2020 US 202063114305 P
(43) Date of publication of application: 20.09.2023
(73) Proprietor: Illumina Cambridge Limited, Cambridge CB21 6DF (GB)
(72) Inventor: ARTIOLI, Gianluca, Andrea, Cambridge, Cambridgeshire CB21 6DF (GB); VON HATTEN, Xavier, Cambridge, Cambridgeshire CB21 6DF (GB)
(74) Representative: Murgitroyd & Company
(86) International application number: PCT/EP2021/081502
(87) International publication number: WO 2022/101401

(56) References cited:
- WO-A1-2016/168386
- US-A1- 2016 122 816
- WANG CHEN ET AL: "DNA-Based Hydrogels Loaded with Au Nanoparticles or Au Nanorods: Thermoresponsive Plasmonic Matrices for Shape-Memory, Self-Healing, Controlled Release, and Mechanical Applications", ACS NANO, vol. 13, no. 3, 1 March 2019 (2019-03-01), US, pages 3424 - 3433, XP055891671, ISSN: 1936-0851, DOI: 10.1021/acsnano.8b09470
- PARK HYE ET AL: "Temperature-Responsive Hydrogel-Coated Gold Nanoshells", GELS, vol. 4, no. 2, 26 March 2018 (2018-03-26), pages 28, XP055892631, DOI: 10.3390/gels4020028
- HONOLD T. ET AL: "Tunable plasmonic surfaces via colloid assembly", JOURNAL OF MATERIALS CHEMISTRY C, vol. 3, no. 43, 1 January 2015 (2015-01-01), GB, pages 11449 - 11457, XP055892639, ISSN: 2050-7526, DOI: 10.1039/C5TC02115D

## Description

### BACKGROUND

Various protocols in biological or chemical research involve performing a large number of controlled reactions on local support surfaces or within predefined reaction chambers. The designated reactions may then be observed or detected and subsequent analysis may help identify or reveal properties of chemicals involved in the reaction. In some examples, the controlled reactions alter charge, conductivity, or some other electrical property, and thus an electronic system may be used for detection. In other examples, the controlled reactions generate fluorescence, and thus an optical system may be used for detection.

### SUMMARY

Plasmonic nanostructures are incorporated into flow cell substrates. In some instances, the plasmonic nanostructures are introduced into a hydrogel of the flow cell, and thus are localized within signal enhancing proximity of an optical label. The optical label, which exhibits fluorescence, is a component of a labeled nucleotide that has been incorporated into a nascent nucleic acid strand during sequencing on the flow cell substrate. Thus, the plasmonic nanostructure is able to enhance the fluorescence of the optical label, which is read as an optical signal.

In other instances, the plasmonic nanostructures are localized within signal quenching proximity of fluorescence signal(s) generated by a substrate resin matrix material. In these instances, the plasmonic nanostructure is able to quench the autofluorescence of the resin matrix material. In these instances, the plasmonic nanostructure functions as a quenching nanostructure, and thus reduces background noise from the resin matrix material.

In still other instances, the plasmonic/quenching nanostructures are capable of both enhancing the optical signals of the optical label and also quenching optical signals from the resin matrix material.

### INTRODUCTION

The present invention provides methods as defined in claim 1 and in claim 3 of increasing a signal to noise ratio during sequencing using a flow cell. The dependent claims set out features of certain embodiments and examples of the methods.

More specifically, in a first aspect, the present invention provides a method for increasing a signal to noise ratio during sequencing in a flow cell, comprising: nanoimprinting a resin matrix material to form a patterned material including depressions separated by interstitial regions; depositing a film of quenching nanostructures onto a surface of the patterned material, the film having a thickness ranging from about 1 nm to about 20 nm; introducing a hydrogel into the depressions; and grafting primers to the hydrogel.

In a second aspect, the present invention provides a method for increasing a signal to noise ratio during sequencing in a flow cell, comprising: incorporating quenching nanostructures into a resin matrix material; patterning the resin matrix material to define a region for an active area surrounded by interstitial regions; introducing a hydrogel into the region; and grafting primers to the hydrogel.

In certain embodiments, the quenching nanostructures are incorporated into the resin matrix material in an amount ranging from about 0.1 wt% to about 10 wt% of a total weight of a mixture of the quenching nanostructures and the resin matrix material.

In certain examples, the region includes a lane and the interstitial regions surround the lane.

In certain examples, the region is a depression; the patterned material defines a plurality of the depressions; and each of the plurality of the depressions is separated by the interstitial regions.

In certain embodiments, the quenching nanostructures are selected from the group consisting of gold nanostructures, silver nanostructures, tin nanostructures, rhodium nanostructures, ruthenium nanostructures, palladium nanostructures, osmium nanostructures, iridium nanostructures, platinum nanostructures, chromium nanostructures, copper nanostructures, gallium arsenide nanostructures, doped silicon nanostructures, aluminum nanostructures, magnesium nanostructures, silver and gold composite nanostructures, and combinations thereof.

The application also describes flow cells and methods of preparing flow cells. The methods of preparing flow cells include applying a hydrogel to a surface of a substrate of the flow cell; grafting primers to the applied hydrogel; and before or after grafting the primers, introducing plasmonic nanostructures to the applied hydrogel.

In one example, the plasmonic nanostructures are functionalized with an alkyne, which covalently attaches to a free azide group of the hydrogel.

In another example, the plasmonic nanostructures are functionalized with an azide, which covalently attaches to an alkyne of the hydrogel.

In a further example, the plasmonic nanostructures are functionalized with a first member of a binding pair, which interacts with a second member of the binding pair that is attached to the hydrogel. In an example, the first member and the second member include a NiNTA ligand and a histidine tag, or streptavidin and biotin, or a spytag and a spycatcher, or maleimide and cysteine, or N-hydroxysuccinimide ester and an amine, or an aldehyde and a hydrazine, or an amine and an activated carboxylate, or an amine and N-hydroxysuccinimide ester, or a thiol and an alkylating reagent, or a phosphoramidite and a thioether.

In some example flow cells, the surface of the substrate includes depressions separated by interstitial regions, and wherein the method further comprises removing the hydrogel from the interstitial regions prior to grafting the primers and prior to introducing the plasmonic nanostructures.

In some examples, the surface of the substrate includes a lane surrounded by interstitial regions, and the method further involves removing the hydrogel from the interstitial regions prior to grafting the primers and prior to introducing the plasmonic nanostructures.

The plasmonic nanostructures may each have a solid structure, a hollow structure, or a core-shell structure.

The application also describes a flow cell comprising a base support; a patterned material over the base support, the patterned material including a resin matrix material, the patterned material defining a region for an active area, the region being surrounded by interstitial regions; a hydrogel in the region, the hydrogel including plasmonic structures; and a primer attached to the hydrogel.

### BRIEF DESCRIPTION OF THE DRAWINGS

Features of examples of the present disclosure will become apparent by reference to the following detailed description and drawings, in which like reference numerals correspond to similar, though perhaps not identical, components. For the sake of brevity, reference numerals or features having a previously described function may or may not be described in connection with other drawings in which they appear.
Fig. 1A through Fig. 1E depict two examples of a method disclosed herein;
Fig. 2A is a top view of an example of a flow cell;
Fig. 2B is an enlarged, cross-sectional view, taken along the 2B-2B line of Fig. 2A, depicting a flow channel and example substrates formed with either of the example methods shown in Fig. 1A through Fig. 1E;
Fig. 2C is an enlarged, cross-sectional view, taken along the 2C-2C line of Fig. 2A, depicting a flow channel and other example substrates formed with either of the example methods shown in Fig. 1A through Fig. 1E;
Fig. 3A through Fig. 3D depict another example method disclosed herein;
Fig. 4A through Fig. 4C depict yet another example method disclosed herein;
Fig. 5A through Fig. 5C depict still another example method disclosed herein;

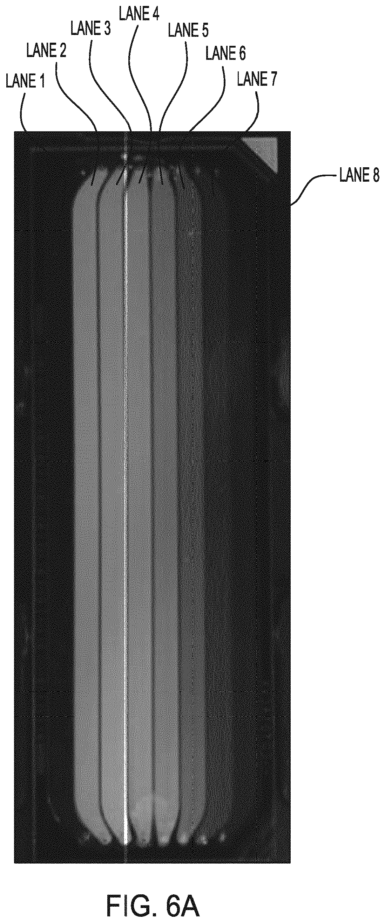

Fig. 6A is a black and white reproduction of an originally colored photograph of a flow cell, where color indicated the presence of a plasmonic nanoparticle solution introduced into some of the lanes of a flow cell, where lane 1 was a control lane with no fluorescent hydrogel (hydrogel with ALEXA FLUOR^{®} 545 dye attached) and no plasmonic nanoparticle solution, lane 2 was a control lane coated with the plasmonic nanoparticle solution but no fluorescent hydrogel, lanes 3-7 were example lanes coated with both the fluorescent hydrogel and with different concentrations of plasmonic nanoparticle solutions, and lane 8 was a control lane coated with the fluorescent hydrogel but no plasmonic nanoparticle solution;
Fig. 6B is a fluorescence scanner image of the flow cell of Fig. 6A collected using a 532 nm green laser (for excitation) and a TAMRA filter after the nanoparticle solutions were flushed from claims 2-7 and after a buffer was introduced, illustrating a fluorescence signal titration in lanes 3-8 and no signals in lanes 1 or 2;
Fig. 6C is a graph depicting the relative intensities corresponding with the fluorescence scanner image of Fig. 6B;
Fig. 6D is a graph illustrating the fluorescence intensity collected using a 532 nm green laser for i) one lane of the flow cell of Fig. 6A including the fluorescent hydrogel (lane 8) but prior to treatment with the nanoparticle solution and after a buffer was introduced, and ii) for lanes 3-7 of the flow cell of Fig. 6A after the nanoparticle solutions were introduced thereto and flushed therefrom and after the buffer was introduced; and
Fig. 7 is a graph illustrating the fluorescence intensity collected using a 488 nm blue laser for i) one lane of a flow cell coated with another fluorescent hydrogel (including ALEXA FLUOR^{®} 488 dye), without nanoparticle solution treatment, and after a buffer was introduced, and ii) for five other lanes of this flow cell after nanoparticle solutions were introduced thereto and flushed therefrom and after the buffer was introduced.

### DETAILED DESCRIPTION

In the examples disclosed herein, nucleotide base calling during sequencing is performed via the detection of fluorescence from an optical label.

In some examples, fluorescence enhancement via plasmonic resonance is achieved through plasmonic nanostructures, which are dispersed throughout a hydrogel of the flow cell used for sequencing. The hydrogel localizes at least some of the plasmonic nanostructures within signal enhancing proximity of an optical label. The optical label, which exhibits fluorescence, is a component of the labeled nucleotide that has been incorporated into a nascent nucleic acid strand during sequencing. By "signal enhancing proximity," it is meant that the plasmonic nanostructure and the optical label are separated by a distance which i) prevents quenching that can occur when the plasmonic nanostructure and the optical label are positioned too close to each other, and ii) increases plasmonic enhancement that can drop significantly at greater distances. The distance corresponding with signal enhancing proximity may range from greater than 0 nm to about 100 nm, but is dependent upon the plasmonic nanostructure (e.g., composition, shape, size) as well as the optical label which exhibits fluorescence upon excitation with a light source. In some instances, the distance corresponding with signal enhancing proximity ranges from about 0.1 nm to about 25 nm, e.g., from about 1 nm to about 20 nm, etc. In one specific example, the distance corresponding with signal enhancing proximity ranges from about 3 nm to about 12 nm.

In other examples, autofluorescence quenching is achieved through plasmonic, or quenching, nanostructures. Some resin matrix materials used in flow cell substrates exhibit autofluorescence during imaging. In some instances, autofluorescence may be the result of optically active component(s) in the resin matrix material migrating towards the surface during heating. Autofluorescence from the resin matrix material can increase the background noise when imaging optical labels of nucleotides that have been incorporated into individual nascent strands during sequencing and that exhibit fluorescence upon excitation with a light source. Increased background noise can decrease signal to noise ratios (SNRs) so that fluorescence, and corresponding signals, from individual optical labels upon excitation with a light source are more difficult to resolve during sequencing. In these examples, the plasmonic nanostructures are dispersed within the resin matrix material or applied as a layer on the resin matrix material, and thus are positioned within "signal quenching proximity" of optically active component(s) in the resin matrix material, adsorbed at the surface of the resin matrix material, or in solution close to the surface of the resin matrix material. By "signal quenching proximity," it is meant that the plasmonic nanostructure and the resin matrix material are close enough to each other (e.g., within 4 nm or less for some nanostructures and optically active component(s)) that the background signal from the resin matrix material is quenched. Quenching the background signals increases the SNR, and thus improves the sequencing primary and secondary metrics. As one example, lower background noise enables bases to be called more accurately, which in turn enables longer sequencing runs.

### Definitions

It is to be understood that terms used herein will take on their ordinary meaning in the relevant art unless specified otherwise. Several terms used herein and their meanings are set forth below.

The singular forms "a", "an", and "the" include plural referents unless the context clearly dictates otherwise.

The terms comprising, including, containing and various forms of these terms are synonymous with each other and are meant to be equally broad.

The terms top, bottom, lower, upper, adjacent, on, etc. are used herein to describe the flow cell and/or the various components of the flow cell. It is to be understood that these directional terms are not meant to imply a specific orientation, but are used to designate relative orientation between components. The use of directional terms should not be interpreted to limit the examples disclosed herein to any specific orientation(s).

The terms first, second, etc. also are not meant to imply a specific orientation or order, but rather are used to distinguish one component from another.

An "acrylamide monomer" is a monomer with the structure 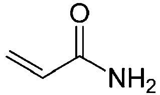 or a monomer including an acrylamide group. Examples of the monomer including an acrylamide group include azido acetamido pentyl acrylamide: 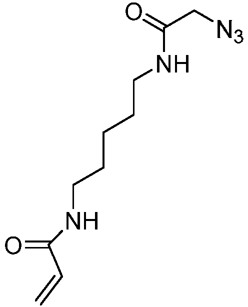 and N-isopropylacrylamide: 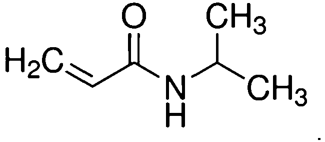 Other acrylamide monomers may be used.

The term "active area" refers to the region of a substrate where a reaction can be carried out. During fabrication of the flow cell, the active area may include a hydrogel that is capable of attaching primers that can participate in nucleic acid template amplification. In the final flow cell, the active area may include the hydrogel with the primers attached thereto. In some instances, the active area also includes plasmonic nanostructures dispersed throughout the hydrogel. The primers in the active area may be used to generate nucleic acid templates that are to be sequenced, and during sequencing, the nucleic acid templates enable the incorporation of nucleotides labeled with an optical label. The optical labels exhibit fluorescence (upon excitation with a light source), and this fluorescence can be enhanced by the plasmonic nanostructures dispersed throughout the hydrogel.

The term "activation," as used herein, refers to a process that generates reactive groups at the surface of a base support or an outermost layer of a multi-layered structure or a film of quenching nanostructures. Activation may be accomplished using silanization or plasma ashing. It is to be understood that activation may be performed in any of the methods disclosed herein. When activation is performed, a silanized layer or -OH groups (from plasma ashing) are present to covalently attach the hydrogel to the underlying support or layer or film.

An "aldehyde," as used herein, is an organic compound containing a functional group with the structure -CHO, which includes a carbonyl center (i.e., a carbon double-bonded to oxygen) with the carbon atom also bonded to hydrogen and an R group, such as an alkyl or other side chain. The general structure of an aldehyde is:

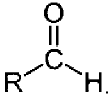

As used herein, "alkyl" refers to a straight or branched hydrocarbon chain that is fully saturated (i.e., contains no double or triple bonds). The alkyl group may have 1 to 20 carbon atoms. Example alkyl groups include methyl, ethyl, propyl, isopropyl, butyl, isobutyl, tertiary butyl, pentyl, hexyl, and the like. As an example, the designation "C1-4 alkyl" indicates that there are one to four carbon atoms in the alkyl chain, i.e., the alkyl chain is selected from the group consisting of methyl, ethyl, propyl, iso-propyl, n-butyl, isobutyl, sec-butyl, and t-butyl.

As used herein, "alkenyl" refers to a straight or branched hydrocarbon chain containing one or more double bonds. The alkenyl group may have 2 to 20 carbon atoms. Example alkenyl groups include ethenyl, propenyl, butenyl, pentenyl, hexenyl, and the like.

As used herein, "alkyne" or "alkynyl" refers to a straight or branched hydrocarbon chain containing one or more triple bonds. The alkynyl group may have 2 to 20 carbon atoms.

As used herein, "aryl" refers to an aromatic ring or ring system (i.e., two or more fused rings that share two adjacent carbon atoms) containing only carbon in the ring backbone. When the aryl is a ring system, every ring in the system is aromatic. The aryl group may have 6 to 18 carbon atoms. Examples of aryl groups include phenyl, naphthyl, azulenyl, and anthracenyl.

An "amino" functional group refers to an -NRₐR_{b} group, where Rₐ and R_{b} are each independently selected from hydrogen (e.g., 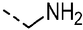 ), C1-6 alkyl, C2-6 alkenyl, C2-6 alkynyl, C3-7 carbocycle, C6-10 aryl, 5-10 membered heteroaryl, and 5-10 membered heterocycle, as defined herein.

As used herein, the term "attached" refers to the state of two things being joined, fastened, adhered, connected or bound to each other, either directly or indirectly. For example, a nucleic acid can be attached to a polymeric hydrogel by a covalent or non-covalent bond. A covalent bond is characterized by the sharing of pairs of electrons between atoms. A non-covalent bond is a physical bond that does not involve the sharing of pairs of electrons and can include, for example, hydrogen bonds, ionic bonds, van der Waals forces, hydrophilic interactions and hydrophobic interactions.

An "azide" or "azido" functional group refers to -N₃.

As used herein, "carbocycle" means a non-aromatic cyclic ring or ring system containing only carbon atoms in the ring system backbone. When the carbocycle is a ring system, two or more rings may be joined together in a fused, bridged or spiro-connected fashion. Carbocycles may have any degree of saturation, provided that at least one ring in a ring system is not aromatic. Thus, carbocycles include cycloalkyls, cycloalkenyls, and cycloalkynyls. The carbocycle group may have 3 to 20 carbon atoms. Examples of carbocycle rings include cyclopropyl, cyclobutyl, cyclopentyl, cyclohexyl, cyclohexenyl, 2,3-dihydro-indene, bicyclo[2.2.2]octanyl, adamantyl, and spiro[ 4.4]nonanyl.

As used herein, the term "carboxylic acid" or "carboxyl" as used herein refers to -COOH.

As used herein, "cycloalkylene" means a fully saturated carbocycle ring or ring system that is attached to the rest of the molecule via two points of attachment.

As used herein, "cycloalkenyl" or "cycloalkene" means a carbocycle ring or ring system having at least one double bond, wherein no ring in the ring system is aromatic. Examples include cyclohexenyl or cyclohexene and norbornenyl or norbornene. Also as used herein, "heterocycloalkenyl" or "heterocycloalkene" means a carbocycle ring or ring system with at least one heteroatom in ring backbone, having at least one double bond, wherein no ring in the ring system is aromatic.

As used herein, "cycloalkynyl" or "cycloalkyne" means a carbocycle ring or ring system having at least one triple bond, wherein no ring in the ring system is aromatic. An example is cyclooctyne. Another example is bicyclononyne. Also as used herein, "heterocycloalkynyl" or "heterocycloalkyne" means a carbocycle ring or ring system with at least one heteroatom in ring backbone, having at least one triple bond, wherein no ring in the ring system is aromatic.

As used herein, the term "depression" refers to a discrete concave feature defined in a substrate and having a surface opening that is at least partially surrounded by interstitial region(s) of the substrate. Depressions can have any of a variety of shapes at their opening in a surface including, as examples, round, elliptical, square, polygonal, star shaped (with any number of vertices), etc. The cross-section of a depression taken orthogonally with the surface can be curved, square, polygonal, hyperbolic, conical, angular, etc. As examples, the depression can be a well or two interconnected wells.

The term "each," when used in reference to a collection of items, is intended to identify an individual item in the collection, but does not necessarily refer to every item in the collection. Exceptions can occur if explicit disclosure or context clearly dictates otherwise.

The term "epoxy" (also referred to as a glycidyl or oxirane group) as used herein refers to

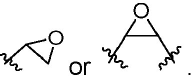

As used herein, the term "flow cell" is intended to mean a vessel having a flow channel where a reaction can be carried out, an inlet for delivering reagent(s) to the flow channel, and an outlet for removing reagent(s) from the flow channel. In some examples, the flow cell accommodates the detection of the reaction that occurs in the flow cell. For example, the flow cell can include one or more transparent surfaces allowing for the optical detection of arrays, optically labeled molecules, or the like.

As used herein, a "flow channel" or "channel" may be an area defined between two bonded components, which can selectively receive a liquid sample, reagents, etc. In some examples, the flow channel may be defined between two substrates, and thus may be in fluid communication with the active areas of each of the substrates. In other examples, the flow channel may be defined between a substrate and a lid, and thus may be in fluid communication with the active areas of one substrate.

As used herein, "heteroaryl" refers to an aromatic ring or ring system (i.e., two or more fused rings that share two adjacent atoms) that contain(s) one or more heteroatoms, that is, an element other than carbon, including but not limited to, nitrogen, oxygen and sulfur, in the ring backbone. When the heteroaryl is a ring system, every ring in the system is aromatic. The heteroaryl group may have 5-18 ring members.

As used herein, "heterocycle" means a non-aromatic cyclic ring or ring system containing at least one heteroatom in the ring backbone. Heterocycles may be joined together in a fused, bridged or spiro-connected fashion. Heterocycles may have any degree of saturation provided that at least one ring in the ring system is not aromatic. In the ring system, the heteroatom(s) may be present in either a non-aromatic or aromatic ring. The heterocycle group may have 3 to 20 ring members (i.e., the number of atoms making up the ring backbone, including carbon atoms and heteroatoms). In some examples, the heteroatom(s) are O, N, or S.

The term "hydrazine" or "hydrazinyl" as used herein refers to a - NHNH₂ group.

As used herein, the term "hydrazone" or "hydrazonyl" as used herein refers to a 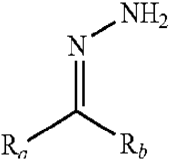 group in which Rₐ and R_{b} are each independently selected from hydrogen, C1-6 alkyl, C2-6 alkenyl, C2-6 alkynyl, C3-7 carbocycle, C6-10 aryl, 5-10 membered heteroaryl, and 5-10 membered heterocycle, as defined herein.

As used herein, "hydroxy" or "hydroxyl" refers to an -OH group.

As used herein, the term "interstitial region" refers to an area, e.g., of a substrate that separates depressions or surrounds a lane. As an example, an interstitial region can separate one depression of an array from another depression of the array. As another example, an interstitial region can separate one lane of a flow cell from another lane of a flow cell. The depressions and lanes that are separated from each other can be discrete, i.e., lacking physical contact with each other. In many examples, the interstitial region is continuous, whereas the depressions or lanes are discrete, for example, as is the case for a plurality of depressions or lanes defined in or on an otherwise continuous surface. The separation provided by an interstitial region can be partial or full separation. Interstitial regions may have a surface material that differs from the surface material of the depressions or lane. For example, depressions and lanes can have the polymeric hydrogel and primers therein, and the interstitial regions can be free of both the polymeric hydrogel and primers.

"Nitrile oxide," as used herein, means a "RₐC≡N⁺O⁻" group in which Rₐ is defined herein. Examples of preparing nitrile oxide include *in situ* generation from aldoximes by treatment with chloramide-T or through action of base on imidoyl chlorides [RC(Cl)=NOH] or from the reaction between hydroxylamine and an aldehyde.

"Nitrone," as used herein, means a 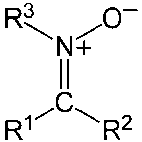 group in which R¹, R², and R³ may be any of the Rₐ and R_{b} groups defined herein, except that R³ is not hydrogen (H).

As used herein, a "nucleotide" includes a nitrogen containing heterocyclic base, a sugar, and one or more phosphate groups. Nucleotides are monomeric units of a nucleic acid sequence. In RNA, the sugar is a ribose, and in DNA, the sugar is a deoxyribose, i.e. a sugar lacking a hydroxyl group that is present at the 2' position in ribose. The nitrogen containing heterocyclic base (i.e., nucleobase) can be a purine base or a pyrimidine base. Purine bases include adenine (A) and guanine (G), and modified derivatives or analogs thereof. Pyrimidine bases include cytosine (C), thymine (T), and uracil (U), and modified derivatives or analogs thereof. The C-1 atom of deoxyribose is bonded to N-1 of a pyrimidine or N-9 of a purine. A nucleic acid analog may have any of the phosphate backbone, the sugar, or the nucleobase altered. Examples of nucleic acid analogs include, for example, universal bases or phosphate-sugar backbone analogs, such as peptide nucleic acid (PNA). A "labeled nucleotide" is a nucleotide that has at least an optical label attached thereto. Examples of optical labels include any dye that is capable of emitting fluorescence in response to an excitation wavelength.

"Plasmonic nanostructures" include any independent structure capable of exhibiting plasmon resonance.

The term "polymeric hydrogel" refers to a semi-rigid polymer that is permeable to liquids and gases. The polymeric hydrogel can swell when liquid (e.g., water) is taken up and that can contract when liquid is removed, e.g., by drying. While a hydrogel may absorb water, it is not water-soluble.

As used herein, the term "primer" is defined as a single stranded nucleic acid sequence (e.g., single stranded DNA). Some primers, referred to herein as amplification primers, serve as a starting point for template amplification and cluster generation. Other primers, referred to herein as sequencing primers, serve as a starting point for DNA synthesis. The 5' terminus of the primer may be modified to allow a coupling reaction with a functional group of a polymeric hydrogel. The primer length can be any number of bases long and can include a variety of non-natural nucleotides. In an example, the sequencing primer is a short strand, ranging from 10 to 60 bases, or from 20 to 40 bases.

"Quenching nanostructures" are plasmonic nanostructures which, due to an energy transfer, minimize wavelengths emitted by an optically active component when in quenching proximity of the optically active component. For example, if the optically active component is in direct contact with a quenching nanostructure transferring energy at the wavelength emitted by the optically active component, fluorescence will be quenched.

A "thiol" functional group refers to -SH.

As used herein, the terms "tetrazine" and "tetrazinyl" refer to six-membered heteroaryl group comprising four nitrogen atoms. Tetrazine can be optionally substituted.

"Tetrazole," as used herein, refer to five-membered heterocyclic group including four nitrogen atoms. Tetrazole can be optionally substituted.

### Plasmonic or Quenching Nanostructures

As set forth herein, plasmonic nanostructures include any independent structure capable of exhibiting plasmon resonance. Plasmon resonance is the phenomenon where the electrons in the material surface layer are excited by photons of incident light with a certain angle of incidence, and then propagate parallel to the material surface. The surfaces of plasmonic nanostructures can strongly confine an electromagnetic field through its coupling to the propagating or localized surface plasmons. This interaction is associated with a large enhancement of the local electrical field, which in turn can enhance the excitation and emission rates and decrease the lifetimes of excited states of fluorescence emitters. This results in an amplified fluorescence signal and may also improve resistance to photobleaching.

Also as set forth herein, quenching nanostructures are nanostructures which, due to an energy transfer, minimize wavelengths emitted by an optically active component when in quenching proximity of the optically active component. As such, quenching nanostructures decrease the autofluorescence intensity of the noise from the optically active component when in quenching proximity of the optically active component.

Any material capable of plasmon resonance, referred to herein as a "plasmonic material", may be used as the plasmonic nanostructures. Certain nanostructures capable of plasmonic resonance are also capable of autofluorescence quenching. Several metals (e.g., gold, silver, tin, rhodium, ruthenium, palladium, osmium, iridium, platinum, copper, aluminum, etc.), doped semi-metals (e.g., doped silicon), direct bandgap semiconductors (e.g., gallium arsenide), and metal composites are capable of plasmon resonance. Metal composites may include two or more of the metals listed above. As examples, a two-metal composite includes silver and gold and a three-metal composite includes silver, gold, and platinum. In any of the examples set forth herein, the plasmonic nanostructures or the quenching nanostructures may be selected from the group consisting of a gold nanostructure, a silver nanostructure, a tin nanostructure, a rhodium nanostructure, a ruthenium nanostructure, a palladium nanostructure, an osmium nanostructure, an iridium nanostructure, a platinum nanostructure, a chromium nanostructure, a copper nanostructure, a gallium arsenide nanostructure, a doped silicon nanostructure, an aluminum nanostructure, a magnesium nanostructure, a silver and gold composite nanostructure, and combinations thereof.

In an example, the plasmonic nanostructures or quenching nanostructures are spherical nanoparticles. In another example, the plasmonic nanostructures or quenching nanostructure are non-spherical nanoparticles, such as cubes, triangular prisms, rod shaped, platelets, cage-like (e.g., non-spherical, hollow particles having a porous shell), tubes, etc. In still another example, the plasmonic nanostructures or quenching nanostructure are irregularly shaped nanoparticles. The morphology of the plasmonic nanostructures may affect the magnitude of the fluorescence enhancement in some of the examples disclosed herein. For example, spherical nanoparticles, nanoplatelets, and nanocubes may magnify fluorescence enhancement more than nanotubes.

The plasmonic nanostructures or quenching nanostructures may each have a solid structure, a hollow structure, or a core-shell structure. The core-shell structure has one material as the core and another material as the shell at least partially encapsulating the core. In some examples, two different plasmonic materials are used as the core and shell. In other examples, the core is a plasmonic material and the shell is a non-plasmonic material. Some examples of suitable shell materials include silica, metal oxides, such as alumina, titania, and tantalum oxides, proteins, such as bovine serum albumin, and organic polymers that are transparent to the wavelengths used during sequencing, such as poly(methyl methacrylate) (PMMA), poly(lactic acid) (PLA), and poly(methyl acrylate) (PMA). The non-plasmonic material does not interfere with the plasmonic resonance of the core. One specific example of a core-shell structure includes a silver and gold composite core at least partially encapsulated by a silica core.

The dimensions of the plasmonic nanostructure or quenching nanostructure may vary depending upon its shape. In the examples disclosed herein, the largest dimension (e.g., diameter, length, median, etc.) of the plasmonic nanostructure or quenching nanostructure is on the nanoscale, and thus ranges from about 1 nm to less than 1000 nm. In some examples, the nanostructures are nanoparticles having a diameter of greater than or equal to 1 nm, 2 nm, 3 nm, 4 nm, 5 nm, 6 nm, 7 nm, 8 nm, 9 nm, 10 nm, 20 nm, 30 nm, 40 nm, 50 nm, 60 nm, 70 nm, 80 nm, 90 nm, or greater than or equal to 100 nm. The size of the plasmonic nanostructures may affect the magnitude of the fluorescence enhancement in some of the examples disclosed herein. More particularly, plasmonic nanostructure(s) with different sizes resonate at different wavelengths. To maximize fluorescent enhancement, the nanostructure resonation wavelength may be considered. For example, modeling may be used to predict the optical properties of a nanostructure of a given size and shape in order to target nanostructures that will resonate at a desired wavelength. In an example, modeling for spherical nanoparticles can be performed by the Mie theory, using Maxwell's equations for light scattering. The chemical structure of the fluorophore having its fluorescence enhanced may also affect the magnitude of the enhancement in some of the examples disclosed herein.

In some examples, the plasmonic nanostructures are functionalized so that they can attach to the hydrogel.

In one example, the plasmonic nanostructures include functional groups that can covalently link to functional groups of the hydrogel. As examples, the plasmonic nanostructure is functionalized with an alkyne (e.g., dibenzocyclooctyne), which covalently attaches to a free azide group of the hydrogel; or the plasmonic nanostructures are functionalized with an azide, which covalently attaches to an alkyne (e.g., a dialkyne) of the hydrogel; or the plasmonic nanostructure is functionalized with an epoxy, which covalently attaches to a free amine group of the hydrogel. Other covalent linkages between the plasmonic nanostructures and the hydrogel are also possible, including those obtained through nucleophilic substitution reactions (e.g., between a nucleophilic group and a nucleofuge group). Some specific examples include those involving an aldehyde and a hydrazine, or an amine and an activated carboxylate (e.g., N-hydroxysuccinimide ester), or a thiol and an alkylating reagent, or a phosphoramidite and a thioether.

In other examples, the plasmonic nanostructures are capable of non-covalently binding to the hydrogel. For example, the plasmonic nanostructure is functionalized with a first member of a binding pair, which interacts with a second member of the binding pair that is attached to the hydrogel. In example binding pairs, the first member and the second member respectively include a NiNTA (nickel- nitrilotriacetic acid) ligand and a histidine tag, or streptavidin or avidin and biotin, or a spytag and a spycatcher.

### Methods and Flow Cells for Plasmonic Enhancement

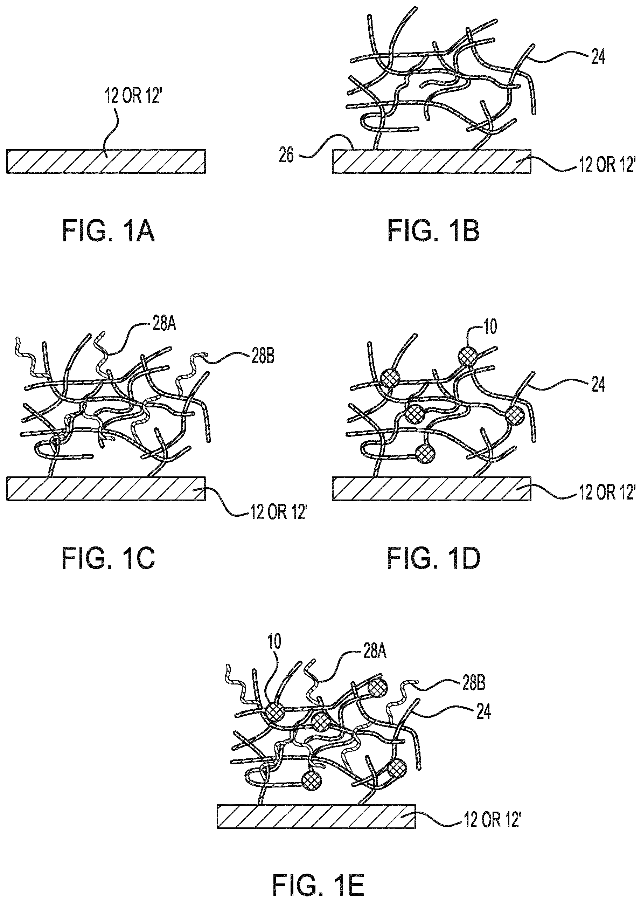

Fig. 1A through Fig. 1E depict two different examples of a method for making a flow cell substrate that can enhance the fluorescence to be read as optical signals during sequencing. One example includes Fig. 1A through Fig. 1C and Fig. 1E. Another example includes Fig. 1A, Fig. 1B, Fig. 1D and Fig. 1E. In each of these examples, plasmonic nanostructures are incorporated into the hydrogel of the flow cell substrate. The methods generally include applying a hydrogel to a surface of a substrate, grafting primers to the applied hydrogel, and before or after grafting the primers, introducing plasmonic nanostructures to the applied hydrogel.

The flow cell substrate is a single layer structure or a multi-layered structure that supports one or more active areas. In Fig. 1A, the substrate is a single layer structure 12. Examples of suitable single layer structures 12 include epoxy siloxane, glass, modified or functionalized glass, plastics (including acrylics, polystyrene and copolymers of styrene and other materials, polypropylene, polyethylene, polybutylene, polyurethanes, polytetrafluoroethylene (such as TEFLON^{®} from Chemours), cyclic olefins/cyclo-olefin polymers (COP) (such as ZEONOR^{®} from Zeon), polyimides, etc.), nylon (polyamides), ceramics/ceramic oxides, silica, fused silica, or silica-based materials, aluminum silicate, silicon and modified silicon (e.g., boron doped p+ silicon), silicon nitride (Si₃N₄), silicon oxide (SiO₂), tantalum pentoxide (Ta₂O₅) or other tantalum oxide(s) (TaOₓ), hafnium oxide (HfO₂), carbon, metals, inorganic glasses, or the like.

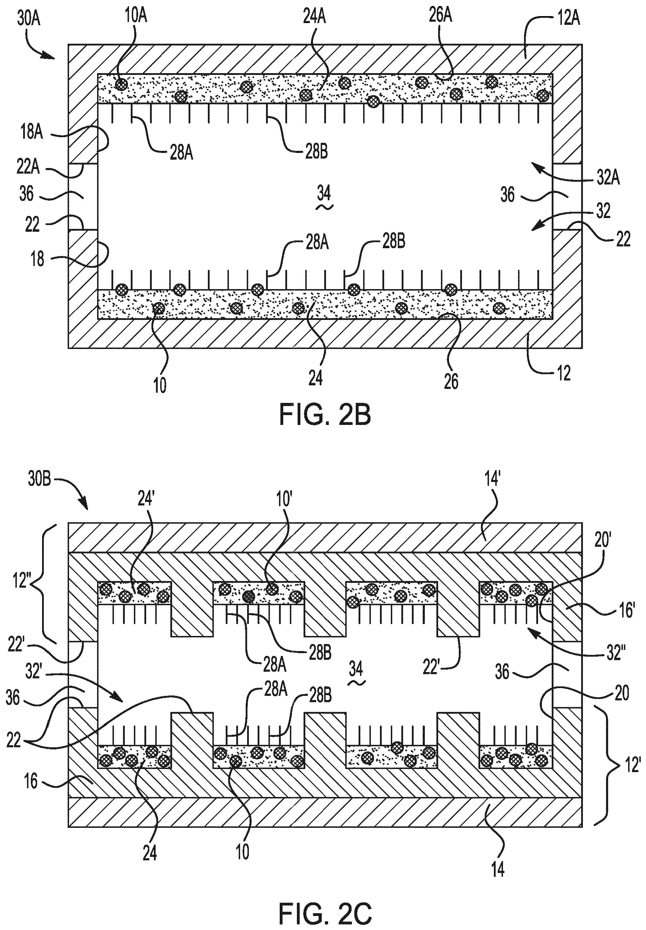

In other examples, the methods shown in Fig. 1A through Fig. 1E may utilize a multi-layered structure 12' (also referred to as a multi-layered substrate 12'). An example of the multi-layered structure 12' is shown in Fig. 2C. Some examples of the multi-layered structure 12' include glass or silicon, with a coating layer of tantalum pentoxide or another oxide that is transparent to the light used in optical imaging. Other examples of the multi-layered substrate 12' may include a silicon-on-insulator (SOI) substrate. With specific reference to Fig. 2C, still other examples of the multi-layered structure 12' include an underlying base support 14 having a patterned material 16 thereon.

In an example, the patterned material 16 may be an inorganic oxide that is selectively applied to the support 14 via vapor deposition, aerosol printing, or inkjet printing. Examples of suitable inorganic oxides include tantalum oxide (e.g., Ta₂O₅), aluminum oxide (e.g., Al₂O₃), silicon oxide (e.g., SiO₂), hafnium oxide (e.g., HfO₂), etc.

As another example, the patterned material 16 may be a resin matrix material that is applied to the support 14 and then patterned. Suitable deposition techniques include chemical vapor deposition, dip coating, dunk coating, spin coating, spray coating, puddle dispensing, ultrasonic spray coating, doctor blade coating, aerosol printing, screen printing, microcontact printing, etc. Suitable patterning techniques include photolithography, nanoimprint lithography (NIL), stamping techniques, embossing techniques, molding techniques, microetching techniques, printing techniques, etc. Some examples of suitable resins include a polyhedral oligomeric silsesquioxane-based resin, a non- polyhedral oligomeric silsesquioxane epoxy resin, a poly(ethylene glycol) resin, a polyether resin (e.g., ring opened epoxies), an acrylic resin, an acrylate resin, a methacrylate resin, an amorphous fluoropolymer resin (e.g., CYTOP^{®} from Bellex), and combinations thereof.

As used herein, the term "polyhedral oligomeric silsesquioxane" (commercially available under the tradename "POSS" from Hybrid Plastics) refers to a chemical composition that is a hybrid intermediate (e.g., RSiO_{1.5}) between that of silica (SiO₂) and silicone (R₂SiO). An example of polyhedral oligomeric silsesquioxane can be that described in Kehagias et al., Microelectronic Engineering 86 (2009), pp. 776-778, to which further reference should be made. In an example, the composition is an organosilicon compound with the chemical formula [RSiO_{3/2}]ₙ, where the R groups can be the same or different. Example R groups for POSS include epoxy, azide/azido, a thiol, a poly(ethylene glycol), a norbornene, a tetrazine, acrylates, and/or methacrylates, or further, for example, alkyl, aryl, alkoxy, and/or haloalkyl groups. The resin composition disclosed herein may comprise one or more different cage or core structures as monomeric units. The average cage content can be adjusted during the synthesis, and/or controlled by purification methods, and a distribution of cage sizes of the monomeric unit(s) may be used in the examples disclosed herein.

In an example, the substrate 12, 12' (whether single or multi-layered) may be round and have a diameter ranging from about 2 mm to about 300 mm, or may be a rectangular sheet or panel having its largest dimension up to about 10 feet (~ 3 meters). In an example, the substrate 12, 12' is a wafer having a diameter ranging from about 200 mm to about 300 mm. Wafers may subsequently be diced to form an individual flow cell substrate. In another example, the substrate 12, 12' is a die having a width ranging from about 0.1 mm to about 10 mm. While example dimensions have been provided, it is to be understood that a substrate 12, 12' with any suitable dimensions may be used. For another example, a panel may be used that is a rectangular support, which has a greater surface area than a 300 mm round wafer. Panels may subsequently be diced to form individual flow cells.

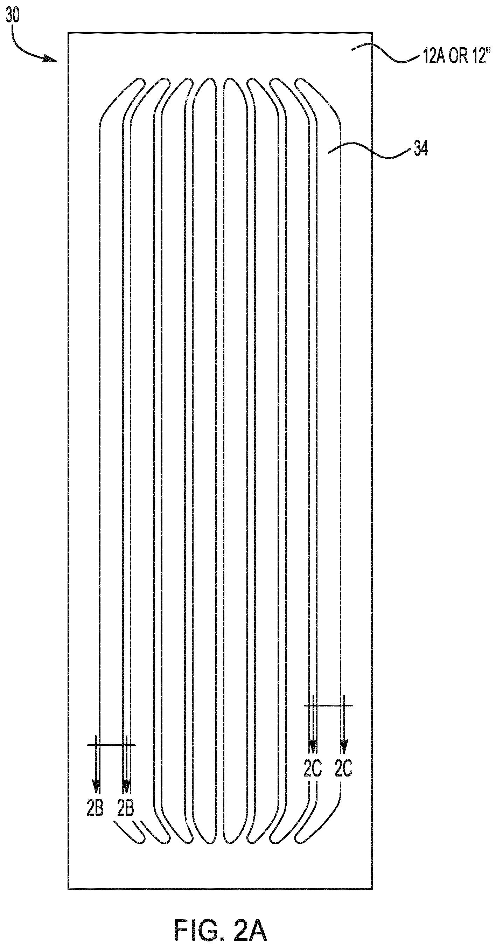

In Fig. 1A, the substrate is depicted as a single layer structure 12 with a flat surface and without any particular architecture. It is to be understood, however, that the substrate 12 or 12' includes some architecture where the active area(s) are formed. In some examples, the architecture of the substrate 12 or 12' includes a single lane 18 (see Fig. 2B) where the active area is formed. In these examples, the single lane 18 is surrounded by interstitial regions 22. In other examples, the architecture of the substrate 12, 12' includes depressions 20 where the active areas are formed (see Fig. 2C). In these examples, the depressions 20 are separated by interstitial regions 22. The single lane 18 or depressions 20 may be formed in a single layer substrate 12, or may be formed in the outermost layer of a multi-layered structure 12'. The single lane 18 or depressions 20 may be defined via etching, imprinting, nanoimprint lithography, or another suitable technique, depending upon the material(s) of the substrate 12, 12'. The architecture of the substrate 12, 12' will be described in more detail in reference to Fig. 2A, Fig. 2B and Fig. 2C.

As shown in Fig. 1B, each example of the method includes applying a hydrogel 24 to the surface 26 of the substrate 12, 12'. The surface 26 of the substrate 12, 12' may include the single lane 18 or the depressions 20, and the interstitial regions 22.

The hydrogel 24 may be any gel material that can swell when liquid is taken up and can contract when liquid is removed, e.g., by drying. In an example, the hydrogel 24 includes an acrylamide copolymer, such as poly(N-(5-azidoacetamidylpentyl)acrylamide-co-acrylamide, PAZAM. PAZAM and some other forms of the acrylamide copolymer are represented by the following structure (I): 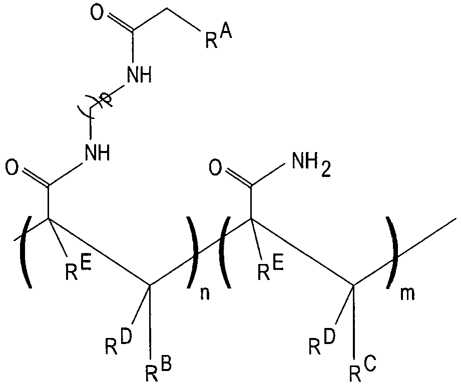 wherein:
R^{A} is selected from the group consisting of azido, optionally substituted amino, optionally substituted alkenyl, optionally substituted alkyne, halogen, optionally substituted hydrazone, optionally substituted hydrazine, carboxyl, hydroxy, optionally substituted tetrazole, optionally substituted tetrazine, nitrile oxide, nitrone, sulfate, and thiol;
R^{B} is H or optionally substituted alkyl;
R^{C}, R^{D}, and R^{E} are each independently selected from the group consisting of H and optionally substituted alkyl;
each of the -(CH₂)ₚ- can be optionally substituted;
p is an integer in the range of 1 to 50;
n is an integer in the range of 1 to 50,000; and
m is an integer in the range of 1 to 100,000.

One of ordinary skill in the art will recognize that the arrangement of the recurring "n" and "m" features in structure (I) are representative, and the monomeric subunits may be present in any order in the polymer structure (e.g., random, block, patterned, or a combination thereof).

The molecular weight of PAZAM and other forms of the acrylamide copolymer may range from about 5 kDa to about 1500 kDa or from about 10 kDa to about 1000 kDa, or may be, in a specific example, about 312 kDa.

In some examples, PAZAM and other forms of the acrylamide copolymer are linear polymers. In some other examples, PAZAM and other forms of the acrylamide copolymer are lightly cross-linked polymers.

In other examples, the hydrogel 24 may be a variation of the structure (I). In one example, the acrylamide unit may be replaced with N,N dimethylacrylamide ( 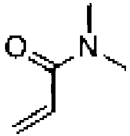 ). In this example, the acrylamide unit in structure (I) may be replaced with 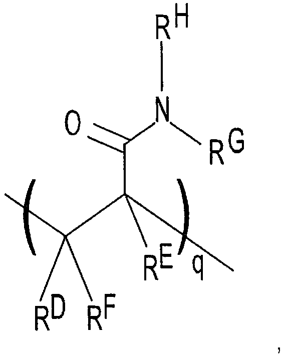 where R^{D}, R^{E}, and R^{F} are each H or a C1-C6 alkyl, and R^{G} and R^{H} are each a C1-C6 alkyl (instead of H as is the case with the acrylamide). In this example, q may be an integer in the range of 1 to 100,000. In another example, the N,N-dimethylacrylamide may be used in addition to the acrylamide unit. In this example, structure (I) may include 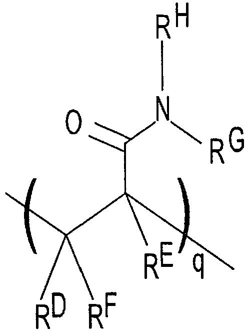 in addition to the recurring "n" and "m" features, where R^{D}, R^{E}, and R^{F} are each H or a C1-C6 alkyl, and R^{G} and R^{H} are each a C1-C6 alkyl. In this example, q may be an integer in the range of 1 to 100,000.

As another example of the hydrogel 24, the recurring "n" feature in structure (I) may be replaced with a monomer including a heterocyclic azido group having structure (II): 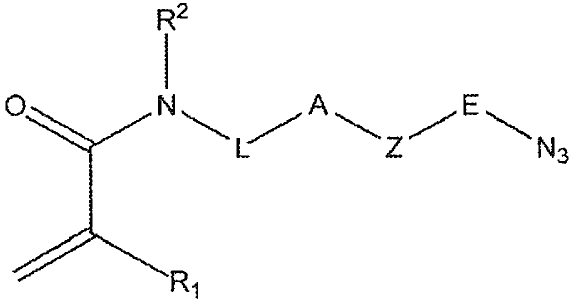 wherein R¹ is H or a C1-C6 alkyl; R₂ is H or a C1-C6 alkyl; L is a linker including a linear chain with 2 to 20 atoms selected from the group consisting of carbon, oxygen, and nitrogen and 10 optional substituents on the carbon and any nitrogen atoms in the chain; E is a linear chain including 1 to 4 atoms selected from the group consisting of carbon, oxygen and nitrogen, and optional substituents on the carbon and any nitrogen atoms in the chain; A is an N substituted amide with an H or a C1-C4 alkyl attached to the N; and Z is a nitrogen containing heterocycle. Examples of Z include 5 to 10 carbon-containing ring members present as a single cyclic structure or a fused structure. Some specific examples of Z include pyrrolidinyl, pyridinyl, or pyrimidinyl.

With the hydrogel 24 examples previously described herein, any of R^{A}, NH₂, and/or N₃ may attach to the plasmonic nanostructures 10 (see Fig. 1D), depending upon the functionalization at the surface of the plasmonic nanostructures 10. Some of the R^{A} and/or N₃ groups may alternatively attach oligonucleotide primers 28A, 28B (see Fig. 1C). Additionally, a binding pair member or another functional group for plasmonic nanostructure 10 attachment may be introduced into the hydrogel 24, e.g., as part of a monomer used in polymerization, or in a grafting process after hydrogel 24 formation, or in a chemical modification reaction.

It is to be understood that other hydrogels 24 may be used, as long as they are functionalized to graft oligonucleotide primers 28A, 28B thereto and to attach plasmonic nanostructures 10 thereto. Some examples of suitable the hydrogel 24 include functionalized polysilanes, such as norbornene silane, azido silane, alkyne functionalized silane, amine functionalized silane, maleimide silane, or any other polysilane having functional groups that can attach the desired plasmonic nanostructures 10 and oligonucleotide primers 28A, 28B. Other examples of suitable hydrogels 24 include those having a colloidal structure, such as agarose; or a polymer mesh structure, such as gelatin; or a cross-linked polymer structure, such as polyacrylamide polymers and copolymers, silane free acrylamide (SFA), or an azidolyzed version of SFA. Examples of suitable polyacrylamide polymers may be synthesized from acrylamide and an acrylic acid or an acrylic acid containing a vinyl group, or from monomers that form [2+2] photo-cycloaddition reactions. Still other examples of suitable polymeric hydrogels include mixed copolymers of acrylamides and acrylates. A variety of polymer architectures containing acrylic monomers (e.g., acrylamides, acrylates etc.) may be utilized in the examples disclosed herein, such as branched polymers, including star polymers, star-shaped or star-block polymers, dendrimers, and the like. For example, the monomers (e.g., acrylamide, acrylamide containing the catalyst, etc.) may be incorporated, either randomly or in block, into the branches (arms) of a star-shaped polymer.

The hydrogel 24 may be formed using any suitable copolymerization process. To introduce the hydrogel 24 to the surface 26, a mixture of the hydrogel 24 may be generated and then applied to the substrate 12 or 12'. In one example, the hydrogel 24 may be present in a mixture (e.g., with water or with ethanol and water). The mixture may then be applied to the substrate surface 26 using spin coating, or dipping or dip coating, or flow of the material under positive or negative pressure, or another suitable technique. These types of techniques blanketly deposit the hydrogel 24 on the substrate 12 or 12' (e.g., in the lane 18, in depressions 20, on interstitial regions 22). Other selective deposition techniques (e.g., involving a mask, controlled printing techniques, etc.) may be used to specifically deposit the hydrogel 24 in the lane 18 or in the depressions 20 and not on the interstitial regions 22.

Depending upon the chemistry of the polymeric hydrogel 24, the applied mixture may be exposed to a curing process. In an example, curing may take place at a temperature ranging from room temperature (e.g., about 25°C) to about 95°C for a time ranging from about 1 millisecond to about several days.

The attachment of the hydrogel 24 to the substrate 12 or 12' may be through covalent bonding. In some instances, the underlying substrate 12 or 12' may first be activated, e.g., through silanization or plasma ashing. In one example, silanization involves introducing norbornene silane to the substrate 12 or 12' surface. Covalent linking of the hydrogel 24 to the substrate 12 or 12' is helpful for maintaining the primers 28A, 28B and the plasmonic nanoparticles 10 in the active area throughout the lifetime of the flow cell during a variety of uses.

When the hydrogel 24 is deposited in the lane 18 or in the depressions 20 and on the interstitial regions 22, the method may further include removing the hydrogel 24 from the interstitial regions 22 prior to grafting the primers 28A, 28B and prior to introducing the plasmonic nanoparticles 10. Removal of the hydrogel 24 may involve a polishing process. The polishing process may be performed with a chemical slurry, which can remove the polymeric hydrogel 24 from the interstitial regions 22 without deleteriously affecting the underlying substrate at those regions 22 and while leaving the polymeric hydrogel 24 in the lane 18 or in the depressions 20 at least substantially intact. An example of the chemical slurry may include abrasive particles, a buffer, a chelating agent, a surfactant, and/or a dispersant. Alternatively, polishing may be performed with a solution that does not include the abrasive particles. The chemical slurry or solution may be used in a chemical mechanical polishing system to polish the surface of the interstitial regions 22. As an example, the polishing head may be a Strasbaugh ViPRR II polishing head.

One example of the method proceeds to Fig. 1C, where the primers 28A, 28B are grafted to the hydrogel 24 prior to the introduction of the plasmonic nanoparticles 10. Grafting may involve flow through deposition (e.g., using a temporarily bound lid), dunk coating, spray coating, puddle dispensing, or another suitable method that will attach the primer(s) 28A, 28B to the polymeric hydrogel 24. Each of these example techniques may utilize a primer solution or mixture, which may include the primer(s) 28A, 28B, water, a buffer, and a catalyst. With any of the grafting methods, the primers 28A, 28B react with reactive groups of the polymeric hydrogel 24 and have no affinity for the surrounding substrate 12, 12' (e.g., interstitial regions 22). As such, the primers 28A, 28B selectively graft to the polymeric hydrogel 24.

In an example, the primers 28A, 28B can be immobilized to the polymeric hydrogel 24 by single point covalent attachment at or near the 5' end of the primers 28A, 28B. This attachment leaves i) an adapter-specific portion of the primers 28A, 28B free to anneal to its cognate sequencing-ready nucleic acid fragment and ii) the 3' hydroxyl group free for primer extension. Any suitable covalent attachment may be used for this purpose. Examples of terminated primers that may be used include alkyne terminated primers (e.g., which may attach to an azide surface moiety of the polymeric hydrogel 24), or azide terminated primers (e.g., which may attach to an alkyne surface moiety of the polymeric hydrogel 24).

Specific examples of suitable primers 28A, 28B include P5 and P7 primers used on the surface of commercial flow cells sold by Illumina Inc. for sequencing on HISEQ^{™}, HISEQX^{™}, MISEQ^{™}, MISEQDX^{™}, MINISEQ^{™}, NEXTSEQ^{™}, NEXTSEQDX^{™}, NOVASEQ^{™}, GENOME ANALYZER^{™}, ISEQ^{™}, and other instrument platforms.

After the primers 28A, 28B are grafted (as shown in Fig. 1C), this example of the method proceeds with introducing the plasmonic nanoparticles 10 to the hydrogel 24. This is shown in Fig. 1E.

The plasmonic nanoparticles 10 may be dispersed in water, and then the substrate 12, 12' (in this example with the hydrogel 24 and primers 28A, 28B thereon) may be flushed with the dispersion. Flushing may involve flow through deposition (e.g., using a temporarily bound lid), dunk coating, puddle dispensing, or another suitable method that will introduce the plasmonic nanoparticles 10 to the polymeric hydrogel 24. The nanoparticle dispersion may be allowed to incubate on the substrate 12, 12' so that the plasmonic nanoparticles 10 can attach to the hydrogel 24. The incubation time and the attachment mechanism will depend upon the functionalization of the plasmonic nanoparticles 10 and the hydrogel 24, the concentration of the plasmonic nanoparticles 10 and the hydrogel 24, etc. In an example, the incubation time may range from 2 minutes to 12 hours. In some examples, incubation at room temperature (e.g., about 25°C) ranges from about 5 minutes to about 1 hour, e.g., from about 5 minutes to about 30 minutes, or from about 1 minutes to about 10 minutes, etc.

As a result of the method shown in Fig. 1A through Fig. 1C and Fig. 1E, the hydrogel 24 includes both primers 28A, 28B and plasmonic nanoparticles 10 attached thereto.

Referring back to Fig. 1B, after the hydrogel 24 is applied, another example of the method proceeds to Fig. 1D, where the plasmonic nanoparticles 10 are introduced to the hydrogel 24 prior to the primers 28A, 28B being grafted. The plasmonic nanoparticles 10 may be dispersed in water, and then the substrate 12, 12' (in this example with the hydrogel 24 thereon) may be flushed with the dispersion as described herein.

After the plasmonic nanoparticles 10 are introduced (as shown in Fig. 1D), this example of the method proceeds with grafting the primers 28A, 28B to the hydrogel 24 having the plasmonic nanoparticles 10 attached thereto. This is shown in Fig. 1E. The primers 28A, 28B may be grafted as described in reference to Fig. 1C.

As a result of the method shown in Fig. 1A, Fig. 1B, Fig. 1D and Fig. 1E, the hydrogel 24 includes both primers 28A, 28B and plasmonic nanoparticles 10 attached thereto.

The structure shown in Fig. 1E depicts one example of the active area of the flow cell. This example of the active area includes the hydrogel 24 with the primers 28A, 28B and the plasmonic nanoparticles 10 attached thereto. As mentioned above, the active area may be located in a single lane 18 of the flow cell (see Fig. 2B) or may be located in depressions 20 of the flow cell (see Fig. 2C). Each of these examples of the flow cell will now be described.

A top view of an example of the flow cell 30 is shown in Fig. 2A. As will be discussed in reference to Fig. 2B, some examples of the flow cell 30A include two opposed substrates 12 and 12A, each of which includes the lane 18, 18A supporting the active area 32 and 32A where sequencing can take place. In each of these examples, a flow channel 34 is defined between the substrates 12 and 12A. As will be discussed in reference to Fig. 2C, other examples of the flow cell 30B include two opposed substrates 12' and 12", each of which includes depressions 20, 20' supporting the active areas 32' and 32" where sequencing can take place. In each of these examples, a flow channel 34 is defined between the substrates 12' and 12". In other examples, the flow cell 30 includes one substrate 12 or 12' (supporting the active area(s) 32 or 32') and a lid attached to the substrate 12 or 12'. In these examples, the flow channel 34 is defined between the substrate 12 or 12' and the lid.

In the example shown in Fig. 2A, the flow cell 30 includes multiple flow channels 34. While eight channels 34 are shown, it is to be understood that any number of channels 34 may be included in the flow cell 30 (e.g., a single channel 34, four channels 34, etc.). Each flow channel 34 may be isolated from each other flow channel 34 in a flow cell 30 so that fluid(s) introduced into any particular flow channel 34 does not flow into any adjacent flow channel 34. Reagents for amplification, clustering, sequencing, de-blocking, etc. can be introduced into and removed from the flow channel(s) 34 through input and output ports, respectively.

A portion of the flow channel 34 may be defined in the substrates 12 and 12A or 12' and 12" using any suitable technique that depends, in part, upon the material(s) of the substrates 12 and 12A or 12' and 12". In one example, a portion of the flow channel 34 is etched into each glass substrate (e.g., 12 and 12A). In another example, a portion of the flow channel 34 may be patterned into the material 16 of the multi-layered substrate 12' using photolithography, nanoimprint lithography, etc. A separate material 36 may be applied to the substrate 12 or 12' so that the separate material 36 defines at least a portion of the walls of the flow channel 34.

In an example, the flow channel 34 has a substantially rectangular configuration with rounded ends. The length and width of the flow channel 34 may be smaller, respectively, than the length and width of the substrate 12 or 12' so that a portion of the substrate surface surrounding the flow channel 34 is available for attachment to another substrate 12A or 12" or to a lid. In some instances, the width of each flow channel 34 can be at least about 1 mm, at least about 2.5 mm, at least about 5 mm, at least about 7 mm, at least about 10 mm, or more. In some instances, the length of each flow channel 34 can be at least about 10 mm, at least about 25 mm, at least about 50 mm, at least about 100 mm, or more. The width and/or length of each flow channel 34 can be greater than, less than or between the values specified above. In another example, the flow channel 34 is square (e.g., 10 mm x 10 mm).

The depth of each flow channel 34 can be as small as a few monolayers thick, for example, when microcontact, aerosol, or inkjet printing is used to deposit the separate material 36 that at least partially defines the flow channel walls. In other examples, the depth of each flow channel 34 can be about 1 µm, about 10 µm, about 50 µm, about 100 µm, or more. In an example, the depth may range from about 10 µm to about 100 µm. In another example, the depth is about 5 µm or less. It is to be understood that the depth of each flow channel 34 can also be greater than, less than or between the values specified above. The depth of the flow channel 34 may also vary along the length and width of the flow cell 30B, e.g., when the depressions 20 are included.

Fig. 2B illustrates a cross-sectional view of the flow cell 30A including the lanes 18, 18A. In this example, a portion of the flow channel 34 is defined in each of the single layer substrates 12, 12A. For example, space within the lane 18, 18A that is not occupied by the components of the active area 32, 32A may be considered to be part of the flow channel 34.

The active areas 32, 32A within the lanes 18, 18A of the respective substrates 12, 12A include the hydrogel 24 or 24A on the surfaces 26 or 26A, the primers 28A, 28B attached to the hydrogel 24 or 24A, and the plasmonic nanoparticles 10, 10A attached to the hydrogel 24 or 24A. Each of these active area 32, 32A may be prepared on the substrate 12, 12A in accordance with either of the methods described in reference to Fig. 1A through Fig. 1E, and then the substrates 12, 12A may be attached to one another to form an example of the flow cell 30A. Any suitable separate material 36, such as an adhesive, a radiation-absorbing material that aids in bonding, etc., may be used to bond the substrates 12, 12A together.

Fig. 2C illustrates a cross-sectional view of the flow cell 30B including the depressions 20, 20' defined in the patterned material 16, 16' of the multi-layered substrates 12', 12". As shown in Fig. 2C, the patterned material 16, 16' includes the depressions 20, 20' respectively defined therein, and interstitial regions 22, 22' separating adjacent depressions 20, 20'.

Many different layouts of the depressions 20, 20' may be envisaged, including regular, repeating, and non-regular patterns. In an example, the depressions 20, 20' are disposed in a hexagonal grid for close packing and improved density. Other layouts may include, for example, rectilinear (rectangular) layouts, triangular layouts, and so forth. In some examples, the layout or pattern can be an x-y format of depressions 20, 20' that are in rows and columns. In some other examples, the layout or pattern can be a repeating arrangement of depressions 20, 20' and/or interstitial regions 22, 22'. In still other examples, the layout or pattern can be a random arrangement of depressions 20, 20' and/or interstitial regions 22, 22'. The pattern may include stripes, swirls, lines, triangles, rectangles, circles, arcs, checks, diagonals, arrows, and/or squares.

The layout or pattern of the depressions 20, 20' may be characterized with respect to the density of the depressions 20, 20' (e.g., number of depressions 20, 20') in a defined area. For example, the depressions 20, 20' may be present at a density of approximately 2 million per mm². The density may be tuned to different densities including, for example, a density of about 100 per mm², about 1,000 per mm², about 0.1 million per mm², about 1 million per mm², about 2 million per mm², about 5 million per mm², about 10 million per mm², about 50 million per mm², or more, or less. It is to be further understood that the density of depressions 20, 20' in the patterned material 16, 16' can be between one of the lower values and one of the upper values selected from the ranges above. As examples, a high density array may be characterized as having depressions 20, 20' separated by less than about 100 nm, a medium density array may be characterized as having depressions 20, 20' separated by about 400 nm to about 1 µm, and a low density array may be characterized as having depressions 20, 20' separated by greater than about 1 µm. While example densities have been provided, it is to be understood that any suitable densities may be used. The density of the depressions 20, 20' may depend, in part, on the depth of the depressions 20, 20'. In some instances, it may be desirable for the spacing between depressions 20, 20' to be even greater than the examples listed herein.

The layout or pattern of the depressions 20, 20' may also or alternatively be characterized in terms of the average pitch, or the spacing from the center of the depression 20, 20' to the center of an adjacent depression 20, 20' (center-to-center spacing) or from the left edge of one depression 20, 20' to the right edge of an adjacent depression 20, 20' (edge-to-edge spacing). The pattern can be regular, such that the coefficient of variation around the average pitch is small, or the pattern can be non-regular in which case the coefficient of variation can be relatively large. In either case, the average pitch can be, for example, about 50 nm, about 0.1 µm, about 0.5 µm, about 1 µm, about 5 µm, about 10 µm, about 100 µm, or more or less. The average pitch for a particular pattern of depressions 20, 20' can be between one of the lower values and one of the upper values selected from the ranges above. In an example, the depressions 20, 20' have a pitch (center-to-center spacing) of about 1.5 µm. While example average pitch values have been provided, it is to be understood that other average pitch values may be used.

The size of each depression 20, 20' may be characterized by its volume, opening area, depth, and/or diameter.

Each depression 20, 20' can have any volume that is capable of confining at least some fluid that is introduced into the flow cell 30B. The minimum or maximum volume can be selected, for example, to accommodate the throughput (e.g., multiplexity), resolution, nucleotides, or analyte reactivity expected for downstream uses of the flow cell 30B. For example, the volume can be at least about 1×10⁻³µm³, at least about 1×10⁻² µm³, at least about 0.1 µm³, at least about 1 µm³, at least about 10 µm³, at least about 100 µm³, or more. Alternatively or additionally, the volume can be at most about 1×10⁴ µm³, at most about 1×10³ µm³, at most about 100 µm³, at most about 10 µm³, at most about 1 µm³, at most about 0.1 µm³, or less.

The area occupied by each depression opening can be selected based upon similar criteria as those set forth above for the volume. For example, the area for each depression opening can be at least about 1×10⁻³ µm², at least about 1×10⁻²µm², at least about 0.1 µm², at least about 1 µm², at least about 10 µm², at least about 100 µm², or more. Alternatively or additionally, the area can be at most about 1×10³ µm², at most about 100 µm², at most about 10 µm², at most about 1 µm², at most about 0.1 µm², at most about 1×10⁻² µm², or less. The area occupied by each depression opening can be greater than, less than or between the values specified above.

The depth of each depression 20, 20' can be large enough to house some of the polymeric hydrogel 24, 24A. In an example, the depth may be at least about 0.1 µm, at least about 0.5 µm, at least about 1 µm, at least about 10 µm, at least about 100 µm, or more. Alternatively or additionally, the depth can be at most about 1×10³ µm, at most about 100 µm, at most about 10 µm, or less. In some examples, the depth is about 0.4 µm. The depth of each depression 20, 20' can be greater than, less than or between the values specified above.

In some instances, the diameter or length and width of each depression 20, 20' can be at least about 50 nm, at least about 0.1 µm, at least about 0.5 µm, at least about 1 µm, at least about 10 µm, at least about 100 µm, or more. Alternatively or additionally, the diameter or length and width can be at most about 1×10³ µm, at most about 100 µm, at most about 10 µm, at most about 1 µm, at most about 0.5 µm, at most about 0.1 µm, or less (e.g., about 50 nm). In some examples, the diameter or length and width is about 0.4 µm. The diameter or length and width of each depression 20, 20' can be greater than, less than or between the values specified above.

In the example shown in Fig. 2C, a portion of the flow channel 34 is defined in each of the multi-layer substrates 12', 12". For example, space within the depression 20, 20' that is not occupied by the components of the active area 32', 32" may be considered to be part of the flow channel 34.

The active areas 32', 32" of the respective substrates 12', 12" include the hydrogel 24 or 24' on the surfaces of each depression 20, 20', the primers 28A, 28B attached to the hydrogel 24 or 24', and the plasmonic nanoparticles 10, 10' attached to the hydrogel 24 or 24'. Each of these active area 32', 32" may be prepared on the substrate 12', 12" in accordance with either of the methods described in reference to Fig. 1A through Fig. 1E, and then the substrates 12', 12" may be attached to one another to form another example of the flow cell 30B. Any suitable separate material 36, such as an adhesive, a radiation-absorbing material that aids in bonding, etc., may be used to bond the substrates 12', 12" together.

When examples of the flow cell 30A or 30B are used in sequencing, template strands (not shown) that are to be sequenced may be formed in the active areas 32, 32A or 32', 32" using the primers 28A, 28B attached to the hydrogel 24, 24A. At the outset of template strand formation, library templates may be prepared from any nucleic acid sample (e.g., a DNA sample or an RNA sample). The DNA nucleic acid sample may be fragmented into single-stranded, similarly sized (e.g., < 1000 bp) DNA fragments. The RNA nucleic acid sample may be used to synthesize complementary DNA (cDNA), and the cDNA may be fragmented into single-stranded, similarly sized (e.g., < 1000 bp) cDNA fragments. During preparation, adapters may be added to the ends of any of the fragments. Through reduced cycle amplification, different motifs may be introduced in the adapters, such as sequencing primer binding sites, indices, and regions that are complementary to the primers 28A, 28B in the active areas 32, 32A or 32', 32". In some examples, the fragments from a single nucleic acid sample have the same adapters added thereto. The final library templates include the DNA or cDNA fragment and adapters at both ends. The DNA or cDNA fragment represents the portion of the final library template that is to be sequenced.

A plurality of library templates may be introduced to the flow cell 30A, 30B. Multiple library templates are hybridized, for example, to one of two types of primers 28A, 28B immobilized in the active areas 32, 32A or 32', 32".

Cluster generation may then be performed. In one example of cluster generation, the library templates are copied from the hybridized primers by 3' extension using a high-fidelity DNA polymerase. The original library templates are denatured, leaving the copies immobilized in the active areas 32, 32A or 32', 32". Isothermal bridge amplification or some other form of amplification may be used to amplify the immobilized copies. For example, the copied templates loop over to hybridize to an adjacent, complementary primer, and a polymerase copies the copied templates to form double stranded bridges, which are denatured to form two single stranded strands. These two strands loop over and hybridize to adjacent, complementary primers and are extended again to form two new double stranded loops. The process is repeated on each template copy by cycles of isothermal denaturation and amplification to create dense clonal clusters. Each cluster of double stranded bridges is denatured. In an example, the reverse strand is removed by specific base cleavage, leaving forward template strands. Clustering results in the formation of several template strands immobilized in the active areas 32, 32A or 32', 32" (e.g., across the lane 18, 18A as shown in Fig. 2B or in depressions 20, 20' as shown in Fig. 2C). This example of clustering is referred to as bridge amplification, and is one example of the amplification that may be performed. It is to be understood that other amplification techniques may be used, such as the exclusion amplification (Examp) workflow (Illumina Inc.).

A sequencing primer (not shown) may be introduced that hybridizes to a complementary portion of the sequence of the template strand. This sequencing primer renders the template strand ready for sequencing.

An incorporation mix including labeled nucleotides may then be introduced into the flow cell 30A, 30B, e.g., via an input port. In addition to the labeled nucleotides, the incorporation mix may include water, a buffer, and polymerases. When the incorporation mix is introduced into the flow cell 30A, 30B, the mix enters the flow channel 34, and contacts the active areas 32, 32A or 32', 32" where the template strands are present.

The incorporation mix is allowed to incubate in the flow cell 30A, 30B, and labeled nucleotides are incorporated by respective polymerases into the nascent strands along the template strands. During incorporation, one of the labeled nucleotides is incorporated, by a respective polymerase, into one nascent strand that extends one sequencing primer and that is complementary to one of the template strands. Incorporation is performed in a template strand dependent fashion, and thus fluorescence detection of type of labeled nucleotides added to the nascent strand can be used to determine the sequence of the template strand. Incorporation occurs in at least some of the template strands across the active area(s) 32, 32A or 32', 32" during a single sequencing cycle.

The incorporated labeled nucleotides may include a reversible termination property due to the presence of a 3' OH blocking group, which terminates further sequencing primer extension once the labeled nucleotide has been added. After a desired time for incubation and incorporation, the incorporation mix, including non-incorporated labeled nucleotides, may be removed from the flow cell 30A, 30B during a wash cycle. The wash cycle may involve a flow-through technique, where a washing solution (e.g., buffer) is directed into, through, and then out of flow channel, e.g., by a pump or other suitable mechanism.

The plasmonic nanoparticles 10, 10A are present in the active areas 32, 32A or 32', 32" where the template strands are immobilized, and where the most recently incorporated labeled nucleotide are located. As such, at least some of the plasmonic nanostructures 10, 10A are held within signal enhancing proximity of the optical label after nucleotide incorporation.

Without further incorporation taking place, the most recently incorporated optical-labeled nucleotides can be detected through an imaging event. During the imaging event, an illumination system (not shown) may provide an excitation light to the active areas 32, 32A or 32', 32". The optical (e.g., dye) labels of the incorporated labeled nucleotides emit fluorescence in response to the excitation light. Additionally, because at least some of the plasmonic nanostructures 10, 10A are within signal enhancing proximity of the respective dye labels, the signals from the dye labels can be enhanced through plasmonic resonance.

After imaging is performed, a cleavage mix may then be introduced into the flow cell 30A, 30B. In an example, the cleavage mix is capable of i) removing the 3' OH blocking group from the incorporated nucleotides, and ii) cleaving the dye label from the incorporated nucleotide. Examples of 3' OH blocking groups and suitable de-blocking agents/components in the cleavage mix may include: ester moieties that can be removed by base hydrolysis; allyl-moieties that can be removed with Nal, chlorotrimethylsilane and Na₂S₂O₃ or with Hg(II) in acetone/water; azidomethyl which can be cleaved with phosphines, such as tris(2-carboxyethyl)phosphine (TCEP) or tris(hydroxypropyl)phosphine (THP); acetals, such as tert-butoxy-ethoxy which can be cleaved with acidic conditions; MOM (-CH₂OCH₃) moieties that can be cleaved with LiBF₄ and CH₃CN/H₂O; 2,4-dinitrobenzene sulfenyl which can be cleaved with nucleophiles such as thiophenol and thiosulfate; tetrahydrofuranyl ether which can be cleaved with Ag(I) or Hg(II); and 3' phosphate which can be cleaved by phosphatase enzymes (e.g., polynucleotide kinase). Examples of suitable dye label cleaving agents/components in the cleavage mix may include: sodium periodate, which can cleave a vicinal diol; phosphines, such as tris(2-carboxyethyl)phosphine (TCEP) or tri(hydroxypropyl)phosphine (THP), which can cleave azidomethyl linkages; palladium and THP, which can cleave an allyl; bases, which can cleave ester moieties; or any other suitable cleaving agent.

Additional sequencing cycles may then be performed until the template strands are sequenced.

### Methods and Flow Cells for Quenching

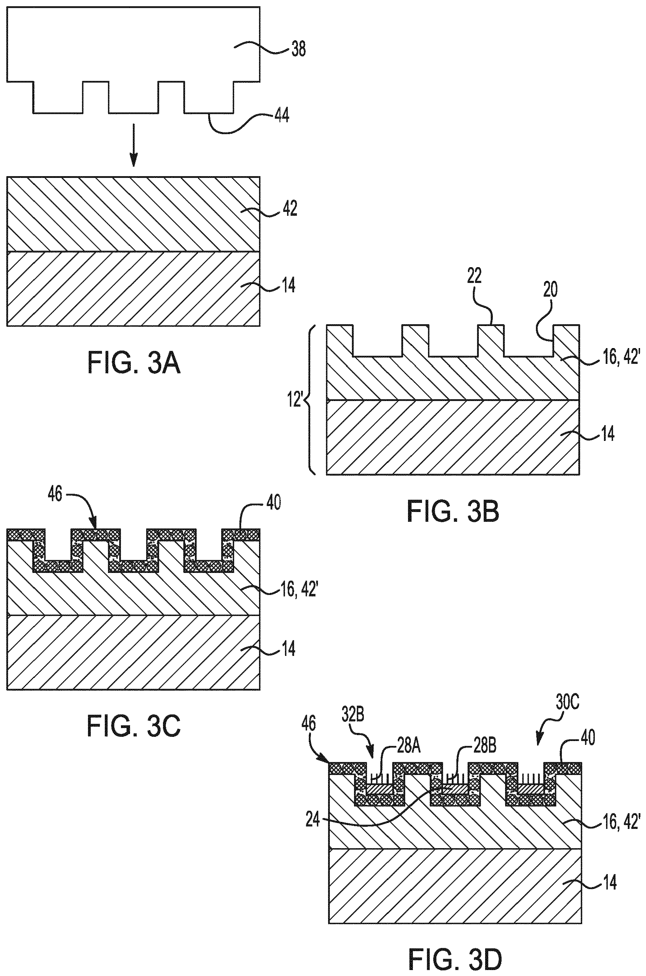

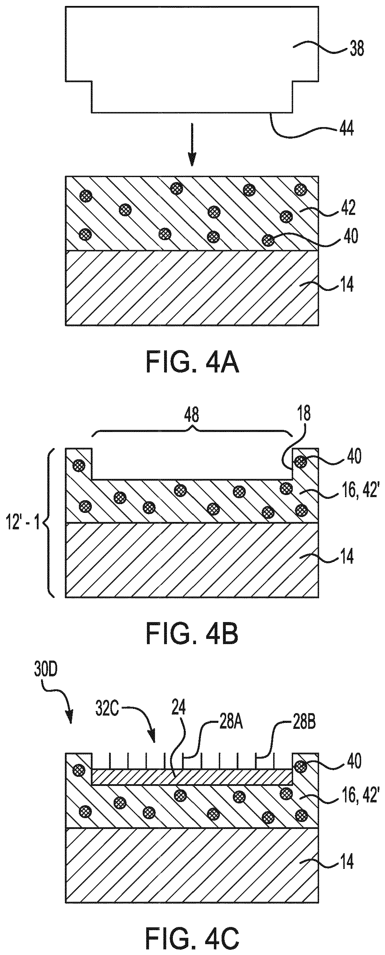

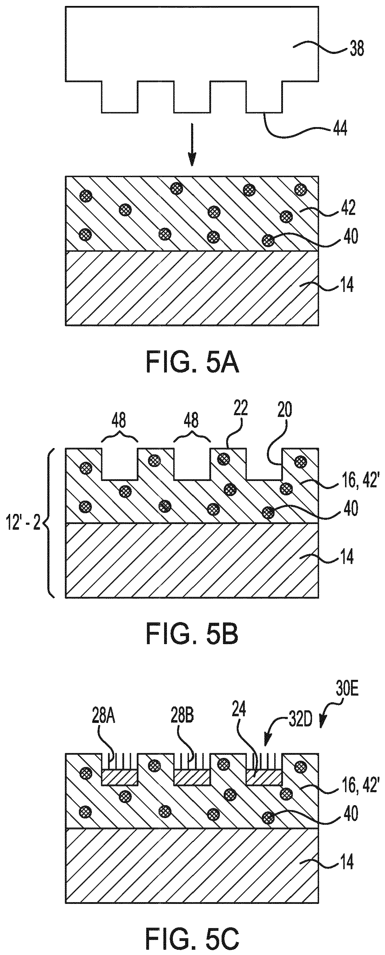

Other examples of the flow cell 30 disclosed herein include quenching nanostructures 40. Portions of each of these flow cells are shown, respectively, at reference numeral 30C in Fig. 3D, 30D in Fig. 4C), and 30E in Fig. 5C. In these examples, the quenching nanostructures 40 are positioned across a surface of the patterned material 16 as shown in the flow cell 30C, or are dispersed throughout the patterned material 16 as shown in the flow cells 30D and 30E. In these example flow cells 30C, 30D, 30E, plasmonic nanostructures 10, 10A are not attached to the hydrogel 24.

As depicted in Fig. 3D, Fig. 4C, and Fig. 5C, each portion of the flow cells 30C, 30D, 30E includes the base support 14, a patterned material 16 over the base support 14, the patterned material 16 including a resin matrix material 42, 42' and a quenching nanostructure 40 dispersed throughout or positioned across a surface of the resin matrix material 42, 42', the patterned material 16 defining a region (e.g., lane 18 or depression 20) for an active area (e.g., 32B, 32C, 32D), the region being surrounded by interstitial regions 22; a hydrogel 24 in the region; and a primer 28A, 28B attached to the hydrogel 24. As such, each of Fig. 3D, Fig. 4C, and Fig. 5C depicts one example of the multi-layer substrate 12', 12'-1, 12'-2 with the active area(s) 32B, 32C, 32D formed thereon. It is to be understood that the final flow cell 30C, 30D, 30E includes a second multi-layer substrate 12', 12'-1, 12'-2 or a lid bonded to the multi-layer substrate 12', 12'-1, 12'-2 and the flow channel 34 defined between the substrates 12', 12'-1, 12'-2 or the substrate 12', 12'-1, 12'-2 and lid.

Some patterned materials 16 exhibit undesirable levels of autofluorescence at excitation wavelengths of interest (e.g., violet excitation wavelengths ranging from about 380 nm to about 450 nm, or blue excitation wavelengths ranging from about 450 nm to about 495 nm, or green excitation wavelengths ranging from about 495 nm to about 570 nm). Autofluorescence from the patterned material 16 can increase the background noise when imaging optical labels of nucleotides that have been incorporated into individual nascent strands formed in the depressions 20 during sequencing. The quenching nanostructures 40 in the flow cells 30C, 30D, 30E are positioned to quench autofluorescence from the patterned material 16. These nanostructures 40 may be selected that quench a target wavelength, e.g., in red, green, blue, violet, or combinations thereof. As such, the quenching nanostructures 40 decrease the background noise, and thus increase the signal to noise ratios (SNRs) so that fluorescence from individual clusters within individual depressions 20 are readily resolvable during sequencing.

Referring now to Fig. 3A through Fig. 3D, an example method for making the flow cell 30C is depicted. This method generally includes nanoimprinting a resin matrix material 42 to form a patterned material 16 including depressions 20 separated by interstitial regions 22; depositing a film 46 of quenching nanostructures 40 onto a surface of the patterned material 16, the film 46 having a thickness ranging from about 1 nm to about 20 nm; introducing a hydrogel 24 into the depressions 20; and grafting primers 28A, 28B to the hydrogel 24.

As depicted in Fig. 3A, this example method utilizes a stack of materials, including the resin matrix material 42 disposed over the base support 14. The resin matrix material 42 and base support 14 may be any of the examples provided herein. The resin matrix material 42 is patterned to form the patterned material 16 (Fig. 3B), and one example of the multi-layer substrate 12' (similar to that shown in Fig. 2C).

In the example shown in Fig. 3A, the resin matrix material 42 is applied to the base support 14. The resin matrix material 42 may be diluted to a viscosity that is suitable for application. Examples of suitable liquid carriers include propylene glycol monomethyl ether acetate (PGMEA), toluene, dimethyl sulfoxide (DMSO), tetrahydrofuran (THF), etc. The diluted resin matrix material 42 may then be applied on the base support 14 using any suitable application technique, which may be manual or automated. As examples, the application of the resin matrix material 42 may be performed using vapor deposition techniques, coating techniques, or the like. Some specific examples include chemical vapor deposition (CVD), spray coating (e.g., ultrasonic spray coating), spin coating, dunk or dip coating, doctor blade coating, puddle dispensing, aerosol printing, screen printing, microcontact printing, inkjet printing, or the like. In one example, spin coating is used.

The technique used to apply the resin matrix material 42 may cause at least some of the liquid carrier to evaporate. After the resin matrix material 42 is applied to the surface of the base support 14, it may be softbaked to remove excess liquid carrier. When performed, the softbake may take place after the resin matrix material 42 is deposited and before a working stamp 38 is positioned therein. The softbake may take place at a lower temperature than is used for curing (e.g., ranging from about 50°C to about 150°C) and for a time ranging from greater than 0 seconds to about 3 minutes. In an example, the softbake time ranges from about 30 seconds to about 2.5 minutes.

The resin matrix material 42 is then patterned, using any suitable patterning technique. In the example shown in Fig. 3A and Fig. 3B, nanoimprint lithography is used to pattern the resin matrix material 42. A nanoimprint lithography mold or working stamp 38 is pressed against the layer of the resin matrix material 42 to create an imprint in the resin matrix material 42. In other words, the resin matrix material 42 is indented or perforated by the protrusions (nanofeatures 44) of the working stamp 38. The resin matrix material 42 may be then be cured with the working stamp 38 in place.

Curing may be accomplished by exposing the applied and nanoimprinted resin matrix material 42 to the actinic radiation, such as ultraviolet (UV) radiation. In one example, the majority of the UV radiation emitted may have a wavelength of about 365 nm. The curing process may include a single UV exposure stage or multiple UV curing stages. After curing, the working stamp 38 is released. The resulting cured resin matrix material 42' and multi-layer substrate 12' is shown in Fig. 3B. The cured resin matrix material 42' has topographic features defined therein, and thus is referred to as the patterned material 16. As shown in Fig. 3B, the patterned material 16 has the depressions 20 defined therein, and each depression 20 is separated by interstitial regions 22.

As shown in Fig. 3C, the method then involves depositing a film 46 of quenching nanostructures 40 onto a surface of the patterned material 16. The quenching nanostructures 40 may be any of the examples set forth herein, and may be deposited using any suitable technique. As examples, the quenching nanostructures 40 may be deposited using electro deposition, spray coating, or chemical vapor deposition.

The quenching nanostructures 40 may be deposited to form a film 46 having a thickness ranging from about 1 nm to about 20 nm. This thickness places the quenching nanostructures 40 within signal quenching proximity of fluorophores in the cured resin matrix material 42'.

As shown in Fig. 3D, the method then includes introducing the hydrogel 24 into the depressions 20, and grafting primers 28A, 28B to the hydrogel 24. Hydrogel 24 application may be performed as described in reference to Fig. 1B and primer 28A, 28B grafting may be performed as described in reference to Fig. 1E. In other examples, the hydrogel 24 may be pre-grafted with primers 28A, 28B, and thus an additional grafting process may not be performed.

Prior to hydrogel 24 application, the film 46 may be activated, e.g., with sulfide norbornene derivatives, or another norbornene derivative that can attach to the quenching nanostructures 40. With sulfide norbornene derivatives, the sulfide can attach to the quenching nanostructures 40 in the film 46 and the norbornene can attach to the subsequently deposited hydrogel 24.

It is to be understood that when polishing is performed during (pre-grafted) hydrogel 24 application, the quenching nanostructures 40 in the film 46 may not be removed from the interstitial regions 22.

This example method generates another example of the active area 32B in each of the depressions 20. This active area 32B includes the hydrogel 24 on the surfaces of each depression 20 and the primers 28A, 28B attached to the hydrogel 24. The quenching nanostructures 40 may or may not be considered part of the active area 32B. As described herein, the quenching nanostructures 40 are positioned to quench signals from the cured resin matrix material 42'. Some of the quenching nanostructures 40 in the film 46 may also be positioned within signal enhancing proximity of optical labels introduced to the depression(s) 20 during sequencing. These quenching nanostructures 40 may have a dual function of quenching cured resin background signals and enhancing optical label signals.

The multi-layer substrate 12' with the film 46 and the active areas 32B shown in Fig. 3D forms a portion of the flow cell 30C, and may be attached (e.g., via separate material 36) to another multi-layer substrate 12' (with the film 46 and the active areas 32B) or to a lid to form the final flow cell 30C. The architecture of the final flow cell 30C may be similar to that shown in Fig. 2C.

Fig. 4A though Fig. 4C and Fig. 5A through 5C show other example methods for making the flow cells 30D and 30E. This example method generally includes incorporating quenching nanostructures 40 into the resin matrix material 42; patterning the resin matrix material 42 to define a region 48 for an active area surrounded by interstitial regions 22; introducing the hydrogel 24 into the region 48; and grafting primers 28A, 28B to the hydrogel 24.

Referring now to Fig. 4A through Fig. 4C, the example method for making the flow cell 30D is depicted. As depicted in Fig. 4A, this example method utilizes a stack of materials, including the base support 14, and the resin matrix material 42 over the base support 14, where the resin matrix material 42 includes the quenching nanostructures 40 dispersed therein.

The base support 14 may be any of the examples provided herein.

The resin matrix material 42 and quenching nanostructures 40 may also be any of the examples provided herein. In this example, the quenching nanostructures 40 are dispersed throughout the resin matrix material 42. Prior to the application of the resin matrix material 42 onto the base support 14, the method may involve incorporating the quenching nanostructures 40 into the resin matrix material 42. In an example, the quenching nanostructures 40 are incorporated into the resin matrix material 42 in an amount ranging from about 0.1 wt% to about 10 wt% of a total weight of a mixture of the quenching nanostructures 40 and the resin matrix material 42. The quenching nanostructures 40 may be added to the resin matrix material 42 to form a mixture, and the mixture may be stirred or otherwise agitated to disperse the quenching nanostructures 40.

In the example shown in Fig. 4A, the resin matrix material 42 having the quenching nanostructures 40 dispersed therein is applied to the base support 14. The resin matrix material 42 having the quenching nanostructures 40 dispersed therein may be diluted with any example of the liquid carrier to a viscosity that is suitable for application. The diluted resin matrix material 42 having the quenching nanostructures 40 dispersed therein may then be applied on the base support 14 using any suitable application technique. After being applied, the resin matrix material 42 having the quenching nanostructures 40 dispersed therein may be softbaked to remove excess liquid carrier as described in reference to Fig. 3A and Fig. 3B.

The resin matrix material 42 having the quenching nanostructures 40 dispersed therein is then patterned, e.g., using nanoimprint lithography, to define the region 48 surrounded by interstitial regions 22, as shown in Fig. 4B. In this example, the region 48 is a lane 18 and the interstitial regions 22 surround the lane 18. As depicted in Fig. 4A, the working stamp 38 in this example method includes nanofeature(s) 44 that is/are a negative replica of the lane(s) 18 that is/are to be formed. When the working stamp 38 is pressed into the resin matrix material 42 having the quenching nanostructures 40 dispersed therein, the resin may be cured as described in reference to Fig. 3A and Fig. 3B. The resulting cured resin matrix material 42' (patterned material 16) and multi-layer substrate 12'-1 is shown in Fig. 4B. As shown in Fig. 4B, the patterned material 16 has the lane 18 defined therein, and the lane 18 is surrounded by interstitial regions 22.

As shown in Fig. 4C, the method then involves introducing the hydrogel 24 into the region 48 (in this instance the lane 18), and grafting primers 28A, 28B to the hydrogel 24. Hydrogel 24 application may be performed as described in reference to Fig. 1B and primer 28A, 28B grafting may be performed as described in reference to Fig. 1E. In other examples, the hydrogel 24 may be pre-grafted with primers 28A, 28B, and thus an additional grafting process may not be performed.

This example method generates another example of the active area 32C in the lane 18. This active area 32C includes the hydrogel 24 on the surfaces of the lane 18 and the primers 28A, 28B attached to the hydrogel 24. In this example, the quenching nanostructures 40 are not considered part of the active area 32C.

The multi-layer substrate 12'-1 with the active area 32C shown in Fig. 4C forms a portion of the flow cell 30D, and may be attached (e.g., via separate material 36) to another multi-layer substrate 12'-1 or to a lid to form the final flow cell 30D. The architecture of the final flow cell 30D may be similar to that shown in Fig. 2B.

Referring now to Fig. 5A through Fig. 5C, the example method for making the flow cell 30E is depicted. As depicted in Fig. 5A, this example method utilizes a stack of materials, including the base support 14, and the resin matrix material 42 over the base support 14, where the resin matrix material 42 includes the quenching nanostructures 40 dispersed therein.

The base support 14 may be any of the examples provided herein.

The resin matrix material 42 and quenching nanostructures 40 may also be any of the examples provided herein. In this example, the quenching nanostructures 40 are dispersed throughout the resin matrix material 42 as described in reference to Fig. 4A through Fig. 4C.

The resin matrix material 42 having the quenching nanostructures 40 dispersed therein is then patterned, e.g., using nanoimprint lithography, to define the region 48 surrounded by interstitial regions 22, as shown in Fig. 5B. In this example, each region 48 is a depression 20 and the interstitial regions 22 surround the depression 20. As depicted in Fig. 4A, the working stamp 38 in this example method includes nanofeature(s) 44 that is/are a negative replica of the depression(s) 20 that is/are to be formed. When the working stamp 38 is pressed into the resin matrix material 42 having the quenching nanostructures 40 dispersed therein, the resin may be cured as described in reference to Fig. 3A and Fig. 3B. The resulting cured resin matrix material 42' (patterned material 16) and multi-layer substrate 12'-2 is shown in Fig. 5B. As shown in Fig. 5B, the patterned material 16 has a plurality of the depressions 20 defined therein, and each depression 20 is separated by interstitial regions 22.

As shown in Fig. 5C, the method then involves introducing the hydrogel 24 into the region 48 (in this instance the depressions 20), and grafting primers 28A, 28B to the hydrogel 24. Hydrogel 24 application may be performed as described in reference to Fig. 1B and primer 28A, 28B grafting may be performed as described in reference to Fig. 1E. In other examples, the hydrogel 24 may be pre-grafted with primers 28A, 28B, and thus an additional grafting process may not be performed.

This example method generates another example of the active area 32D in each depression 20. This active area 32D includes the hydrogel 24 on the surfaces of each depression 20 and the primers 28A, 28B attached to the hydrogel 24. In this example, the quenching nanostructures 40 are not considered part of the active area 32D.

The multi-layer substrate 12'-2 with the active area 32D shown in Fig. 5C forms a portion of the flow cell 30E, and may be attached (e.g., via separate material 36) to another multi-layer substrate 12'-2 or to a lid to form the final flow cell 30E. The architecture of the final flow cell 30E may be similar to that shown in Fig. 2C.

The examples of the flow cell 30C, 30D, and 30E may be used in a sequencing method as described herein for the flow cells 30A and 30B. Due to the presence of the quenching nanostructures 40, the signal to noise ratio may be increased during sequencing. The plasmonic enhancement described in reference to the flow cells 30A and 30B may or may not occur with the flow cell 30C, 30D, and 30E, and will depend, in part, upon the distance of the quenching nanostructures 40 from the optical label of each incorporated labeled nucleotide during imaging.

### Flow Cells for Plasmonic Enhancement and for Quenching

Still other examples of the flow cell 30 disclosed herein are hybrids. One example is a hybrid of the flow cell 30B shown in Fig. 2C and of the flow cell 30C shown in Fig. 3D. This example includes plasmonic nanostructures 10 in the hydrogel 24 and quenching nanostructures 40 in the film 46 formed over the patterned material 16. Another example is a hybrid of the flow cell 30A shown in Fig. 2B and of the flow cell 30D shown in Fig. 4C. Still another example is a hybrid of the flow cell 30B shown in Fig. 2C and of the flow cell 30E shown in Fig. 5C. These examples include plasmonic nanostructures 10 in the hydrogel 24 and quenching nanostructures 40 in the patterned material 16.

The hybrid examples may be used in a sequencing method as described herein for the flow cells 30A and 30B. Due to the presence of both the plasmonic nanostructures 10 and the quenching nanostructures 40, the signals from the optical labels of each incorporated labeled nucleotide may be enhanced and signals from the cured resin matrix material 42' may be quenched. As such, the signal to noise ratio may be increased during sequencing.

To further illustrate the present disclosure, examples are given herein. It is to be understood that these examples are provided for illustrative purposes and are not to be construed as limiting the scope of the present disclosure.

### NON-LIMITING WORKING EXAMPLES

### Example 1

This example was performed to demonstrate fluorescence enhancement from plasmonic nanoparticles incorporated into the hydrogel of the flow cell.

In this example, a patterned flow cell with eight flow channels (8 cm long) was used. The flow channels are referred to as lanes 1-8 in this example. Each flow channel included depressions formed in a patterned resin material.

The first and second flow channels (lanes 1 and 2) were control flow channels and were not coated with any hydrogel. The third through eighth flow channels (lanes 3-8) were coated with fluorescent PAZAM (i.e., PAZAM with ALEXA FLUOR^{®} 545 dye grafted thereto).

Five different plasmonic nanoparticle solutions, with different nanoparticle concentrations, were prepared with silver nanocubes (~75 nm) in water. The concentrations of the solutions are shown in Table 1.

**TABLE 1**

| **Nanoparticle Solution ID** | **Nanoparticle Concentration** |
|---|---|
| NPS 1 | 1 mg/mL |
| NPS 2 | 0.75 mg/mL |
| NPS 3 | 0.5 mg/mL |
| NPS 4 | 0.25 mg/mL |
| NPS 5 | 0.1 mg/mL |

100 µL of each solution, NPS 1 - NPS 5, was respectively flushed in lanes 3 - 7 (coated with fluorescent PAZAM). 100 µL of NPS 1 (1 mg/mL) was also flushed in control lane 2 (not coated with fluorescent PAZAM). None of the nanoparticle solutions were introduced into control lane 1 (not coated with fluorescent PAZAM) or control lane 8 (coated with fluorescent PAZAM). A photograph of the flow cell after introduction of the nanoparticle solution was taken, and is reproduced herein in Fig. 6A. In the original color photograph, the lanes coated with the nanoparticle solution were green, and the color was brighter as the nanoparticle concentration was increased. Thus, control lane 2 and lane 3 (each coated with NPS 1 and thus the highest concentration of nanoparticles) exhibited the brightest green color. The colors confirmed the presence of the different nanoparticle solutions, NPS 1 - NPS 5.

A fluorescent scan (532 nm excitation) on a TYPHOON^{®} scanner (Amersham) of the flow cell was performed with the nanoparticle solutions in lanes 2-7. While the results are not reproduced herein, fluorescent signals were observed in lanes 3-7 (with NPS 1 - NPS 5 respectively therein) and in control lane 8 (fluorescent PAZAM but none of the nanoparticle solutions), but were not observed in either control lanes 1 or 2. The results for control lane 2 confirmed that no fluorescence signals emanated from the nanoparticles themselves.

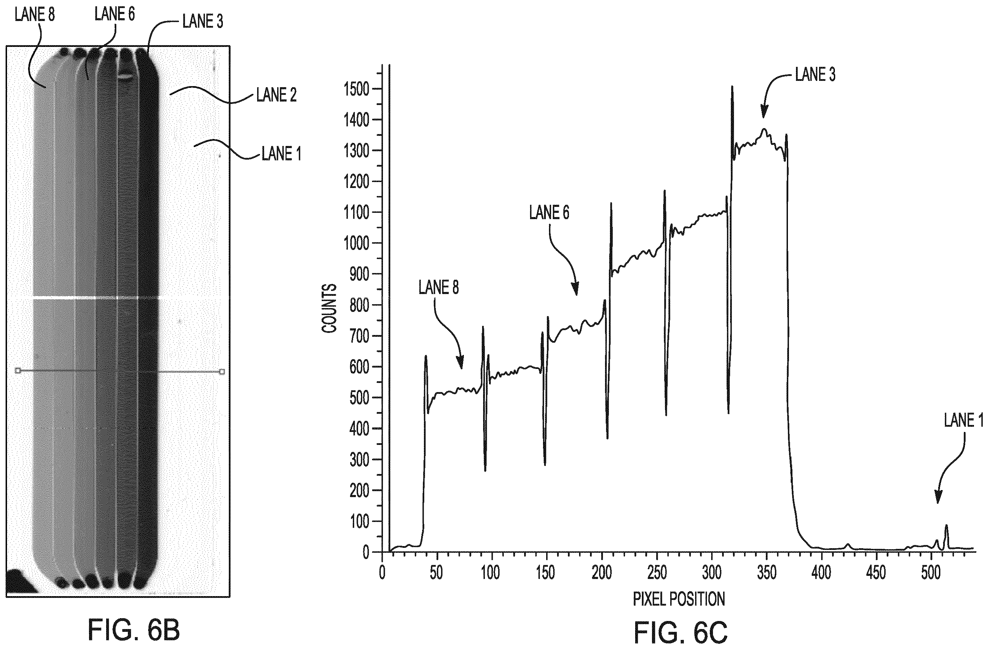

The nanoparticle solutions NPS 1 - NPS 5 were then flushed out of lanes 2-7. A sodium citrate buffer was introduced into each of lanes 1-8, and another fluorescent scan (532 nm excitation) of the flow cell on the TYPHOON^{®} scanner (Amersham) was performed. The image from this scan is reproduced in Fig. 6B, with lane 8 at the left and lane 1 at the right. The relative intensities corresponding with the image of Fig. 6B are shown in the graph in Fig. 6C. As shown in both Fig. 6B and Fig. 6C, no fluorescence signal was observed in the control lanes 1 and 2 without the fluorescent PAZAM. These results confirmed that the nanoparticles that had been introduced into control lane 2 were removed when NPS 1 was flushed therefrom. In contrast, fluorescence signals were observed in lane 8, which included fluorescent PAZAM but no nanoparticle solution, and in each of lanes 3-7, which included fluorescent PAZAM and had been coated with NPS1 - NPS 5. These results demonstrate that the plasmonic nanoparticles did become trapped in the hydrogel in lanes 3-7 prior to the respective nanoparticle solutions being flushed therefrom, because the fluorescent signals in lanes 3-7 increased as the concentration increased (i.e., lane 3 had the strongest signal and lane 7 had the weakest signal). These results demonstrated that the nanoparticles enhanced the fluorescence signals, and that the concentration can affect the magnitude of the enhancement.

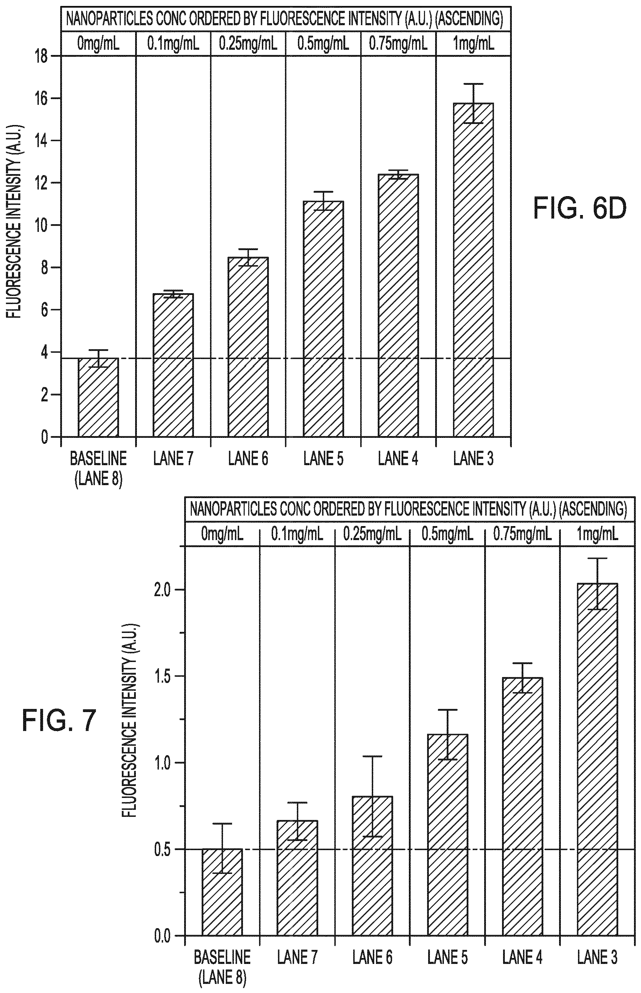

After the fluorescent PAZAM was introduced into lanes 3-8 and prior to the introduction of the nanoparticle solutions, a fluorescent scan (532 nm excitation) on the TYPHOON^{®} scanner of the flow cell was performed. The scan of lane 8 provided a baseline fluorescence intensity measurement for the coated lanes prior to treatment with one of NSP 1 through NSP 5. This baseline measurement is shown in the graph in Fig. 6D.

After nanoparticle solution introduction, incubation, and removal, and after sodium citrate buffer introduction, another fluorescent scan (532 nm excitation) of the flow cell on the TYPHOON^{®} scanner (Amersham) was performed. The fluorescence intensity for lanes 3-7 from the fluorescent scan (532 nm excitation) performed after nanoparticle solution introduction, incubation, and removal, and after sodium citrate buffer introduction is also shown in Fig. 6D.

The results in Fig. 6D show that the larger the concentration of plasmonic nanostructures in the solution introduced into the hydrogel, the larger the fluorescence enhancement. For example, lane 3 exposed to the highest concentration of nanoparticles (1 mg/mL) reached a 4.5x enhancement in green (532 nm) signals compared to the baseline measurement.

### Example 2

To further investigate the effect that nanoparticle concentration can have on the magnitude of the plasmonic nanoparticle enhancement, another flow cell with a different fluorescent hydrogel was prepared and tested.

In this example, a patterned flow cell with eight flow channels (8 cm long) was used. Each flow channel included depressions formed in a patterned resin material. All of the lanes were coated with a second fluorescent PAZAM, which in this example was PAZAM with ALEXA FLUOR^{®} 488 dye grafted thereto.

After the second fluorescent PAZAM was introduced into the lanes, a fluorescent scan (488 nm excitation) on the TYPHOON^{®} scanner of the flow cell was performed. The scan of lane 8 provided a baseline fluorescence intensity measurement for the coated lanes prior to treatment with one of NSP 1 through NSP 5 of Example 1. This baseline measurement is shown in the graph in Fig. 7.

100 µL of each nanoparticle solution from Example 1, NPS 1 - NPS 5, was respectively flushed in lanes 3 - 7 (coated with the second fluorescent PAZAM). The solutions were allowed to incubate for 5 minutes at room temperature before being flushed out of the flow cell. A sodium citrate buffer was introduced into each of lanes 1-8, and another fluorescent scan (488 nm excitation) of the flow cell on the TYPHOON^{®} scanner (Amersham) was performed. The fluorescence intensity for lanes 3-7 are also shown in Fig. 7.

The results in Fig. 7 show that the larger the concentration of plasmonic nanostructures in the solution introduced into the hydrogel, the larger the fluorescence enhancement. For example, lane 3 exposed to the highest concentration of nanoparticles (1 mg/mL) reached a 4.5x enhancement both in blue (488 nm) signals compared to the standard intensity obtained on the same flowcell scanned before the nanoparticle treatment. These results are consistent with the results in green (532 nm) shown in Fig. 6D.

### Example 3

The same type of flow cell from Example 1 was used. In this example, 3 lanes were treated as follows: lane 1 was coated with PAZAM and grafted with P5 and P7 primers; lane 2 was coated with PAZAM, treated with NPS 1 (1 mg/ml) from Example 1 for 5 minutes at room temperature before NPS 1 was removed, and then grafted with P5 and P7 primers; and lane 3 was coated with PAZAM, grafted with P5 and P7 primers, and then treated with NPS 1 (1 mg/ml) from Example 1 for 5 minutes at room temperature before NPS 1 was removed.

Each of lanes 1-3 was then exposed to an ALEXA FLUOR^{™} 545-P5 complement, which hybridized to, and thus labeled, the P5 primers in the lanes. A fluorescent scan (532 nm excitation) of the flow cell on the TYPHOON^{®} scanner (Amersham) was performed. While the results are not reproduced herein, fluorescence enhancement was not observed in lane 1, but was observed in lanes 2 and 3, both of which were treated with the plasmonic nanoparticles. These results indicated that the plasmonic nanoparticles may be introduced to the hydrogel either before or after primer grafting.

### Additional Notes

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure or any reference mentioned herein should be accorded a meaning most consistent with the particular concepts disclosed herein.

Reference throughout the specification to "one example", "another example", "an example", and so forth, means that a particular element (e.g., feature, structure, and/or characteristic) described in connection with the example is included in at least one example described herein, and may or may not be present in other examples. In addition, it is to be understood that the described elements for any example may be combined in any suitable manner in the various examples unless the context clearly dictates otherwise.

It is to be understood that the ranges provided herein include the stated range and any value or sub-range within the stated range, as if such values or sub-ranges were explicitly recited. For example, a range from about 2 mm to about 300 mm, should be interpreted to include not only the explicitly recited limits of from about 2 mm to about 300 mm, but also to include individual values, such as about 40 mm, about 250.5 mm, etc., and sub-ranges, such as from about 25 mm to about 175 mm, etc.

Furthermore, when "about" and/or "substantially" are/is utilized to describe a value, they are meant to encompass minor variations (up to +/- 10%) from the stated value.

While several examples have been described in detail, it is to be understood that the disclosed examples may be modified. Therefore, the foregoing description is to be considered non-limiting.

## Claims

1. A method for increasing a signal to noise ratio during sequencing in a flow cell, comprising:
nanoimprinting a resin matrix material to form a patterned material including depressions separated by interstitial regions;
depositing a film of quenching nanostructures onto a surface of the patterned material, the film having a thickness ranging from about 1 nm to about 20 nm;
introducing a hydrogel into the depressions; and
grafting primers to the hydrogel.

2. The method as defined in claim 1, wherein the quenching nanostructures are selected from the group consisting of gold nanostructures, silver nanostructures, tin nanostructures, rhodium nanostructures, ruthenium nanostructures, palladium nanostructures, osmium nanostructures, iridium nanostructures, platinum nanostructures, chromium nanostructures, copper nanostructures, gallium arsenide nanostructures, doped silicon nanostructures, aluminum nanostructures, magnesium nanostructures, silver and gold composite nanostructures, and combinations thereof.

3. A method for increasing a signal to noise ratio during sequencing in a flow cell, comprising:
incorporating quenching nanostructures into a resin matrix material;
patterning the resin matrix material to define a region for an active area surrounded by interstitial regions;
introducing a hydrogel into the region; and
grafting primers to the hydrogel.

4. The method as defined in claim 3, wherein the quenching nanostructures are incorporated into the resin matrix material in an amount ranging from about 0.1 wt% to about 10 wt% of a total weight of a mixture of the quenching nanostructures and the resin matrix material.

5. The method as defined in claim 3 or claim 4, wherein the quenching nanostructures are selected from the group consisting of gold nanostructures, silver nanostructures, tin nanostructures, rhodium nanostructures, ruthenium nanostructures, palladium nanostructures, osmium nanostructures, iridium nanostructures, platinum nanostructures, chromium nanostructures, copper nanostructures, gallium arsenide nanostructures, doped silicon nanostructures, aluminum nanostructures, magnesium nanostructures, silver and gold composite nanostructures, and combinations thereof.

6. The method as defined in any one of claims 3 to 5, wherein the region includes a lane and the interstitial regions surround the lane.

7. The method as defined in any one of claims 3 to 5, wherein:
the region is a depression;
the patterned material defines a plurality of the depressions; and
each of the plurality of the depressions is separated by the interstitial regions.

## Patentansprüche

1. Ein Verfahren zum Erhöhen eines Signal-Rausch-Verhältnisses während der Sequenzierung in einer Durchflusszelle, das Folgendes beinhaltet:
Nanoprägen eines Harzmatrixmaterials, um ein strukturiertes Material zu bilden, das Vertiefungen umfasst, die durch interstitielle Regionen getrennt sind;
Abscheiden eines Films aus Quencher-Nanostrukturen auf einer Oberfläche des strukturierten Materials, wobei der Film eine Dicke im Bereich von etwa 1 nm bis etwa 20 nm aufweist;
Einführen eines Hydrogels in die Vertiefungen; und
Pfropfen von Primern auf das Hydrogel.

2. Verfahren gemäß Anspruch 1, wobei die Quencher-Nanostrukturen aus der Gruppe ausgewählt sind, die aus Folgendem besteht: Gold-Nanostrukturen, Silber-Nanostrukturen, Zinn-Nanostrukturen, Rhodium-Nanostrukturen, Ruthenium-Nanostrukturen, Palladium-Nanostrukturen, Osmium-Nanostrukturen, Iridium-Nanostrukturen, Platin-Nanostrukturen, Chrom-Nanostrukturen, Kupfer-Nanostrukturen, Galliumarsenid-Nanostrukturen, Nanostrukturen aus dotiertem Silizium, Aluminium-Nanostrukturen, Magnesium-Nanostrukturen, Silber- und Gold-Komposit-Nanostrukturen und Kombinationen davon.

3. Ein Verfahren zum Erhöhen eines Signal-Rausch-Verhältnisses während der Sequenzierung in einer Durchflusszelle, das Folgendes beinhaltet:
Einbringen von Quencher-Nanostrukturen in ein Harzmatrixmaterial;
Strukturieren des Harzmatrixmaterials, um eine Region für einen aktiven Bereich zu definieren, die von interstitiellen Regionen umgeben ist;
Einführen eines Hydrogels in die Region; und
Pfropfen von Primern auf das Hydrogel.

4. Verfahren gemäß Anspruch 3, wobei die Quencher-Nanostrukturen in das Harzmatrixmaterial in einer Menge im Bereich von etwa 0,1 Gew.-% bis etwa 10 Gew.-% eines Gesamtgewichts einer Mischung der Quencher-Nanostrukturen und des Harzmatrixmaterials eingebracht werden.

5. Verfahren gemäß Anspruch 3 oder Anspruch 4, wobei die Quencher-Nanostrukturen aus der Gruppe ausgewählt sind, die aus Folgendem besteht: Gold-Nanostrukturen, Silber-Nanostrukturen, Zinn-Nanostrukturen, Rhodium-Nanostrukturen, Ruthenium-Nanostrukturen, Palladium-Nanostrukturen, Osmium-Nanostrukturen, Iridium-Nanostrukturen, Platin-Nanostrukturen, Chrom-Nanostrukturen, Kupfer-Nanostrukturen, Galliumarsenid-Nanostrukturen, Nanostrukturen aus dotiertem Silizium, Aluminium-Nanostrukturen, Magnesium-Nanostrukturen, Silber- und Gold-Komposit-Nanostrukturen und Kombinationen davon.

6. Verfahren gemäß einem der Ansprüche 3 bis 5, wobei die Region eine Spur umfasst und die interstitiellen Regionen die Spur umgeben.

7. Verfahren gemäß einem der Ansprüche 3 bis 5, wobei:
die Region eine Vertiefung ist;
das strukturierte Material eine Vielzahl der Vertiefungen definiert; und
jede der Vielzahl der Vertiefungen durch die interstitiellen Regionen getrennt ist.

## Revendications

1. Un procédé pour l'augmentation d'un rapport signal sur bruit pendant le séquençage dans une cellule d'écoulement *(flow cell*), comprenant :
la nano-impression d'un matériau de matrice en résine pour former un matériau à motif incluant des enfoncements séparés par des régions interstitielles ;
le dépôt d'un film de nanostructures de trempe sur une surface du matériau à motif, le film ayant une épaisseur allant d'environ 1 nm à environ 20 nm ;
l'introduction d'un hydrogel dans les enfoncements ; et
le greffage d'amorces sur l'hydrogel.

2. Le procédé tel que défini dans la revendication 1, dans lequel les nanostructures de trempe sont sélectionnées dans le groupe constitué de nanostructures d'or, de nanostructures d'argent, de nanostructures d'étain, de nanostructures de rhodium, de nanostructures de ruthénium, de nanostructures de palladium, de nanostructures d'osmium, de nanostructures d'iridium, de nanostructures de platine, de nanostructures de chrome, de nanostructures de cuivre, de nanostructures d'arséniure de gallium, de nanostructures de silicium dopé, de nanostructures d'aluminium, de nanostructures de magnésium, de nanostructures composites d'argent et d'or, et de combinaisons de celles-ci.

3. Un procédé pour l'augmentation d'un rapport signal sur bruit pendant le séquençage dans une cellule d'écoulement (*flow cell*), comprenant :
l'incorporation de nanostructures de trempe dans un matériau de matrice en résine ;
le modelage selon un motif du matériau de matrice en résine pour définir une région pour une zone active entourée par des régions interstitielles ;
l'introduction d'un hydrogel dans la région ; et
le greffage d'amorces sur l'hydrogel.

4. Le procédé tel que défini dans la revendication 3, dans lequel les nanostructures de trempe sont incorporées dans le matériau de matrice en résine en une quantité allant d'environ 0,1 % en poids à environ 10 % en poids d'un poids total d'un mélange des nanostructures de trempe et du matériau de matrice en résine.

5. Le procédé tel que défini dans la revendication 3 ou la revendication 4, dans lequel les nanostructures de trempe sont sélectionnées dans le groupe constitué de nanostructures d'or, de nanostructures d'argent, de nanostructures d'étain, de nanostructures de rhodium, de nanostructures de ruthénium, de nanostructures de palladium, de nanostructures d'osmium, de nanostructures d'iridium, de nanostructures de platine, de nanostructures de chrome, de nanostructures de cuivre, de nanostructures d'arséniure de gallium, de nanostructures de silicium dopé, de nanostructures d'aluminium, de nanostructures de magnésium, de nanostructures composites d'argent et d'or, et de combinaisons de celles-ci.

6. Le procédé tel que défini dans l'une quelconque des revendications 3 à 5, dans lequel la région inclut une voie et les régions interstitielles entourent la voie.

7. Le procédé tel que défini dans l'une quelconque des revendications 3 à 5, dans lequel :
la région est un enfoncement ;
le matériau à motif définit une pluralité des enfoncements ; et
chaque enfoncement de la pluralité des enfoncements est séparé par les régions interstitielles.
